(19) Europäisches Patentamt
European Patent Office
Office européen des brevets

(11) EP 4 059 522 A1

(12) EUROPEAN PATENT APPLICATION

(43) Date of publication:
21.09.2022 Bulletin 2022/38

(21) Application number: 22152108.1

(22) Date of filing: 08.02.2016

(51) International Patent Classification (IPC):
*A61K 47/69* (2017.01) *A61K 31/57* (2006.01)
*A61K 9/00* (2006.01) *A61P 25/08* (2006.01)

(52) Cooperative Patent Classification (CPC):
**A61K 9/0019; A61K 9/08; A61K 9/19; A61K 31/57; A61K 47/40; A61K 47/6951; A61P 25/08**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 06.02.2015 US 201562112943 P

(62) Document number(s) of the earlier application(s) in accordance with Art. 76 EPC:
**16706109.2 / 3 253 418**

(71) Applicant: **Marinus Pharmaceuticals, Inc.**
**Radnor, Pennsylvania 19087-5279 (US)**

(72) Inventors:
- **ZHANG, Mingbao**
**Millwood, 10546 (US)**
- **GLOWAKY, Raymond C.**
**Killingworth, 06419 (US)**

(74) Representative: **Bull, Duncan Richard**
**Kilburn & Strode LLP**
**Lacon London**
**84 Theobalds Road**
**London WC1X 8NL (GB)**

Remarks:
•This application was filed on 18-01-2022 as a divisional application to the application mentioned under INID code 62.
•Claims filed after the date of receipt of the divisional application (Rule 68(4) EPC).

# (54) INTRAVENOUS GANAXOLONE FORMULATIONS AND THEIR USE IN TREATING STATUS EPILEPTICUS AND OTHER SEIZURE DISORDERS

(57) The disclosure provides an injectable ganaxolone formulation comprising ganaxolone, sulfobutyl ether-β-cyclodextrin; and water. The injectable ganaxolone formulation optionally includes a surfactant and a pH modifier. The ganaxolone and sulfobutyl ether-β-cyclodextrin may be in an inclusion complex. The disclosure also provides a lyophilized powder of the ganaxolone/ sulfobutyl ether-β-cyclodextrin formulation that may be reconstituted in water for injection. The disclosure provides a method of treating a patient having a seizure disorder, stroke, or traumatic brain injury, comprising administering an effective amount of the injectable ganaxolone formulation comprising ganaxolone, sulfobutyl ether-β-cyclodextrin; and water. The disclosure also provides combination methods in which the injectable ganaxolone/ sulfobutyl ether-β-cyclodextrin formulation is administered in combination with at least one additional active agent.



Processed by Luminess, 75001 PARIS (FR)

## Description

BACKGROUND

Status epilepticus (SE) is a serious seizure disorder in which the epileptic patient experiences a seizure lasting more than five minutes, or more than one seizure in a five minute period without recovering between seizures. In certain instances convulsive seizures may last days or even weeks. Status epilepticus is treated in the emergency room with conventional anticonvulsants. $GABA_A$ receptor modulators such as benzodiazepines (BZs) are a first line treatment. Patients who fail to respond to BZs alone are usually treated with anesthetics or barbiturates in combination with BZs. About 23-43% of status epilepticus patients who are treated with a benzodiazepine and at least one additional antiepileptic drug fail to respond to treatment and are considered refractory. (Rossetti, A.O. and Lowenstein, D.H., Lancet Neurol. (2011) 10(10): 922-930.) There are currently no good treatment options for these patients. The mortality rate for refractory status epilepticus (RSE) patients is high and most RSE patients do not return to their pre-RSE clinical condition. About 15% of patients admitted to hospital with SE are in a subgroup of RSE patients said to be super-refractory SE (SRSE), in which the patients have continued or recurrent seizures 24 hours or more after the onset of anesthetic therapy. SRSE is associated with high rates of mortality and morbidity. (Shorvon, S., and Ferlisi, M., Brain, (2011) 134(10): 2802-2818.)

Parenteral allopregnanolone has been proposed as a treatment for refractory status epilepticus. (Rogawski, M.A., et al., Epilepsia, (2013) 54: 93-98.) However allopregnanolone has a very short half-life and is metabolized to the active steroid, 5α-dihydroprogesterone, making allopregnanolone a non-ideal drug choice for RSE treatment.

Another serious seizure disorder is PCDH19 female pediatric epilepsy, which affects approximately 15,000-30,000 females in the United States. This genetic disorder is associated with seizures beginning in the early years of life, mostly focal clustered seizures that can last for weeks. The mutation of the PCDH19 gene has been associated with low levels of allopregnanolone, but treatment with allopregnanolone is a non-ideal choice for reasons given above. Currently there are no approved therapies for PCDH19 female pediatric epilepsy.

Thus, there exists the need for additional treatments for seizure disorders such as status epilepticus, refractory status epilepticus, super refractory status epilepticus, and PCDH19 female pediatric epilepsy. This disclosure fulfills this need and provides additional advantages that are described herein.

SUMMARY

The disclosure provides an injectable ganaxolone formulation comprising ganaxolone, sulfobutyl ether-β-cyclodextrin; and water. The ganaxolone and sulfobutyl ether-β-cyclodextrin may be in an inclusion complex. Sulfobutyl ether β-cyclodextrin is marketed under the trade name, CAPTISOL® (Ligand Pharmaceuticals), which contains an average 6-7 sulfobutyl ether groups per cyclodextrin molecule. The disclosure also provides a lyophilized powder of the ganaxolone/ sulfobutyl ether-β-cyclodextrin formulation that may be reconstituted in water for injection.

The disclosure also provides a method of treating a patient having a seizure disorder, stroke, or traumatic brain injury, comprising administering an effective amount of the injectable ganaxolone formulation comprising ganaxolone, sulfobutyl ether-β-cyclodextrin; and water

The disclosure includes methods of treatment in which ganaxolone is the only active agent and methods in which the ganaxolone / sulfobutyl ether-β-cyclodextrin formulation is administered in combination with an additional active agent.

The disclosure includes methods of treatment which include administration schedules for the ganaxolone / sulfobutyl ether-β-cyclodextrin formulation, alone or with at least one additional active agent.

BRIEF DESCRIPTION OF THE DRAWINGS

FIGURE 1. A plot of ganaxolone concentration in solution (mg/ mL), as determined by HPLC, versus CAPTISOL concentration (mg/ mL) provides a 52: 1 weight: weight ratio of CAPTISOL:ganaxolone needed for ganaxolone solubilization.

FIGURE 2. Plasma exposure of ganaxolone in rats infused with ganaxolone-CAPTISOL solution. The ganaxolone: CAPTISOL ratio was 1:60 (w/w).

FIGURE 3. Vehicle, Allopregnanolone (15 mg/kg) or Ganaxolone (12 or 15 mg/kg IV) were administered to rats at 15 min after SE-onset. Average FFT power is shown with baseline values subtracted from subsequent values for each animal before averaging; thus baseline = 0 for each treatment. Each panel (A-F) shows mean ± SEM of EEG power from at the following time points: - 135'= Baseline; -75'= Scopolamine; -45'= Pilocarpine; -15'= Status Epilepticus Onset; 0'= 0-15' post dosing (PD); 15'= 15-30' PD; 30'= 30-60' PD; 60'= 1-2 h PD; 120'= 2-3 h PD; 180'=

3-5 h PD. Vertical dashed line = first post-dosing time point, 0-15'.

FIGURE 4. Vehicle, Allopregnanolone (15 mg/kg) or Ganaxolone (12 or 15 mg/kg IV) were administered to rats at 60 min after SE-onset. Average FFT power is shown with baseline values subtracted from subsequent values for each animal before averaging; thus baseline = 0 for each treatment. Each panel (A-F) shows mean ± SEM of EEG power from at the following time points: - 180'= Baseline; -120'= Scopolamine; -90'= Pilocarpine; -60'= Status Epilepticus Onset; 0'= 0-15' post dosing (PD); 15'= 15-30' PD; 30'= 30-60' PD; 60'= 1-2 h PD; 120'= 2-3 h PD; 180'= 3-5 h PD. Vertical dashed line = first post-dosing time point, 0-15'.

DETAILED DESCRIPTION

DEFINITIONS

Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable. All methods described herein can be performed in a suitable order unless otherwise indicated herein or otherwise clearly contradicted by context. The use of any and all examples, or exemplary language (e.g., "such as"), is intended merely for illustration and does not pose a limitation on the scope of the invention unless otherwise claimed. No language in the specification should be construed as indicating any non-claimed element as essential to the practice of the invention.

The terms "a" and "an" do not denote a limitation of quantity, but rather denote the presence of at least one of the referenced item.

The term "about" is used synonymously with the term "approximately." As one of ordinary skill in the art would understand, the exact boundary of "about" will depend on the component of the composition. Illustratively, the use of the term "about" indicates that values slightly outside the cited values, i.e., plus or minus 0.1% to 10%, which are also effective and safe. Thus compositions slightly outside the cited ranges are also encompassed by the scope of the present claims.

An "active agent" is any compound, element, or mixture that when administered to a patient alone or in combination with another agent confers, directly or indirectly, a physiological effect on the patient. When the active agent is a compound, salts, solvates (including hydrates) of the free compound or salt, crystalline and non-crystalline forms, as well as various polymorphs of the compound are included. Compounds may contain one or more asymmetric elements such as stereogenic centers, stereogenic axes and the like, e.g. asymmetric carbon atoms, so that the compounds can exist in different stereoisomeric forms. These compounds can be, for example, racemates or optically active forms. For compounds with two or more asymmetric elements, these compounds can additionally be mixtures of diastereomers. For compounds having asymmetric centers, it should be understood that all of the optical isomers in pure form and mixtures thereof are encompassed. In addition, compounds with carbon-carbon double bonds may occur in Z- and E-forms, with all isomeric forms of the compounds being included in the present invention. In these situations, the single enantiomers, i.e. optically active forms, can be obtained by asymmetric synthesis, synthesis from optically pure precursors, or by resolution of the racemates. Resolution of the racemates can also be accomplished, for example, by conventional methods such as crystallization in the presence of a resolving agent, or chromatography, using, for example a chiral HPLC column.

The terms "comprising," "including," and "containing" are non-limiting. Other non-recited elements may be present in embodiments claimed by these transitional phrases. Where "comprising," "containing," or "including" are used as transitional phrases other elements may be included and still form an embodiment within the scope of the claim. The open-ended transitional phrase "comprising" encompasses the intermediate transitional phrase "consisting essentially of" and the close-ended phrase "consisting of."

A "bolus dose" is a relatively large dose of medication administered in a short period, for example within 1 to 30 minutes.

$C_{max}$ is the measured concentration of an active concentration in the plasma at the point of maximum concentration.

The term "inclusion complex" is intended to mean a complex between a ganaxolone molecule and a cyclodextrin molecule. A molecule of ganaxolone may be partially inserted into the hydrophobic cavity of one cyclodextrin molecule. In certain non-limiting embodiments the inclusion complex has one ganaxolone molecule and one sulfobutyl ether-β-cyclodextrin molecule, to give a 1:1 ratio between ganaxolone and sulfobutyl ether-β-cyclodextrin.

Infusion administration is a non-oral administration, typically intravenous though other non-oral routes such as epidural administration are included in some embodiments. Infusion administration occurs over a longer period than a bolus administration, for example over a period of at least 15 minutes, at least 30 minutes, at least 1 hour, at least 2 hours, at least 3 hours, or at least 4 hours.

A "patient" is a human or non-human animal in need of medical treatment. Medical treatment includes treatment of an existing condition, such as a disorder or injury. In certain embodiments treatment also includes prophylactic or preventative treatment, or diagnostic treatment

"Reduced irritation" is irritation that is less than irritation that occurs with injectable ganaxolone formulations that are not fully solubilized. The reduced irritation is minimal to mild irritation at the site of injection or at the muscular site which is acceptable to the patient and does not impact unfavorably on patient compliance.

A "therapeutically effective amount" or "effective amount" is that amount of a pharmaceutical agent to achieve a pharmacological effect. The term "therapeutically effective amount" includes, for example, a prophylactically effective amount. An "effective amount" of ganaxolone is an amount needed to achieve a desired pharmacologic effect or therapeutic improvement without undue adverse side effects. The effective amount of ganaxolone will be selected by those skilled in the art depending on the particular patient and the disease. It is understood that "an effective amount" or "a therapeutically effective amount" can vary from subject to subject, due to variation in metabolism of ganaxolone, age, weight, general condition of the subject, the condition being treated, the severity of the condition being treated, and the judgment of the prescribing physician.

"Treat" or "treatment" refers to any treatment of a disorder or disease, such as inhibiting the disorder or disease, e.g., arresting the development of the disorder or disease, relieving the disorder or disease, causing regression of the disorder or disease, relieving a condition caused by the disease or disorder, or reducing the symptoms of the disease or disorder.

## CHEMICAL DESCRIPTION

Ganaxolone (CAS Reg. No. 38398-32-2, 3α-hydroxy, 3β-methyl-5(α-pregnan-20-one) is a synthetic steroid with anti-convulsant activity useful in treating epilepsy and other central nervous system disorders.

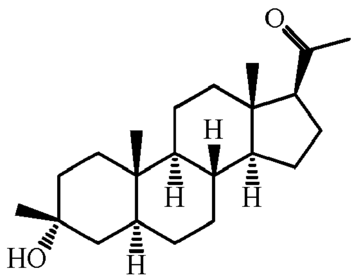

3α-hydroxy, 3β-methyl-5α-pregnan-20-one **Ganaxolone**

Ganaxolone has a relatively long half-life - approximately 20 hours in human plasma following oral administration (Nohria, V. and Giller, E., Neurotherapeutics, (2007) 4(1): 102-105). Furthermore, ganaxolone has a short $T_{max}$, which means that therapeutic blood levels are reached quickly. Thus initial bolus doses (loading doses) may not be required, which represents an advantage over other treatments. Ganaxolone is useful for treating seizures in adult and pediatric epileptic patients.

U.S. Pat. Nos. 5,134,127 and 5,376,645 each to Stella et al. disclose sulfoalkyl ether cyclodextrin derivatives and their use as solubilizing agents for water-insoluble drugs for oral, intranasal or parenteral administration including intravenous and intramuscular administration. Stella et al. disclose an inclusion complex of the water-insoluble drug and the sulfoalkyl ether cyclodextrin derivative, and pharmaceutical compositions containing these inclusion complexes. Examples of sulfoalkyl ether cyclodextrin derivatives disclosed include mono-sulfobutyl ethers of β-cyclodextrin and monosulfopropyl ethers of β-cyclodextrin. CAPTISOL, marketed by Ligand Pharmaceuticals is a sulfobutyl ether β-cyclodextrin with an average 6-7 sulfobutyl ether groups per cyclodextrin molecule. CAPTISOL is sold as an amorphous material and has an average molecular weight of 2163 g/ mole based on 6.5 substitutions per molecule.

## INJECTABLE SUBSTITUTED BETA-CYCLODEXTRIN-GANAXOLONE FORMULATIONS

The disclosure provides injectable substituted β-cyclodextrin ganaxolone formulations, including formulations containing CAPTISOL-ganaxolone inclusion complexes. Injectable substituted β-cyclodextrin ganaxolone formulations disclosed herein include formulations suitable for intramuscular, intravenous, intraarterial, intraspinal, and intrathecal injection. Injectable formulations include parenteral formulations suitable for intravenous infusion.

The disclosure provides injectable substituted β-cyclodextrin ganaxolone formulations containing an inclusion complex of a substituted-β-cyclodextrin, such as CAPTISOL, and ganaxolone, and a pharmaceutically acceptable carrier. In certain embodiments the substituted β-cyclodextrin ganaxolone formulation of the disclosure will be in the form of an

aqueous parenteral or injectable formulation.

Ganaxolone is very poorly soluble in water (< 0.001 mg/ mL) and thus is difficult to formulate as an aqueous injectable. The inventors have found that the water-solubility of ganaxolone may be sufficiently increased to allow it to be formulated as an aqueous injectable by complexing ganaxolone with a substituted β-cyclodextrin, such as CAPTISOL. In effect, the substituted β-cyclodextrin inhibits precipitation of the ganaxolone at the injection site, providing reduced irritation and permitting injection without unacceptable injection-site irritation.

The injectable substituted [β-cyclodextrin ganaxolone formulations provided in this disclosure are aqueous formulations or powder formulations including lyophilized forms, which may be readily dissolved in water to provide an injectable formulation. The disclosure includes embodiments in which the lyophilized ganaxolone powder comprising ganaxolone and sulfobutyl ether-β-cyclodextrin, wherein the formulation is 1.0% to 2.0% wt. ganaxolone.

The disclosure provides injectable substituted β-cyclodextrin ganaxolone formulations containing ganaxolone at a concentration of 0.25 mg/ mL, 0.5 mg/ mL, 1.0 mg/ mL, 1.5 mg/ mL, 2.0 mg/ mL, 2,5 mg/ mL, 3.0 mg/ mL, 3.5 mg/ mL, 4.0 mg/ mL, 4,5 mg/ mL, 5.0 mg/ mL, 5.5 mg/ mL, 6.0 mg/ mL, 6.5 mg/ mL, 7.0 mg/ mL, 7.5 mg/ mL, 8.0 mg/ mL, 8.5 mg/ mL, 9.0 mg/ mL, 10 mg/ mL, 11 mg/ mL, 12 mg/ mL, 13 mg/ mL, or about 15 mg/ mL. All ranges including any two of the foregoing concentrations of substituted β-cyclodextrin as endpoints are also included in the disclosure. For example, the disclosure includes substituted β-cyclodextrin ganaxolone formulations containing from about 0,5mg/ mL to about 15 mg/ mL, about 1.0 mg/ mL to about 10 mg/ mL, about 2.0 mg/ mL to about 8,0 mg/ mL, or about 4,0 mg/ mL to about 8.0 mg/ mg ganaxolone. An embodiment comprising an aqueous injectable ganaxolone/ sulfobutyl ether β-cyclodextrin formulation (e.g. in an inclusion complex) and containing from about 2.0 mg/ mL to about 8.0 mg/ mL ganaxolone is included in this disclosure.

The substituted β-cyclodcxtrm will be in a weight: weight ratio to ganaxolone of about 10:1 to 100: 1, or about 40:1 to about 80:1, or about 52: 1 to about 80:1, or about 52: 1 to about 85:1, or about 55:1 to about 70: 1, or about 55:1 to about 65:1 or about 55:1. The ratio of substituted β-cyclodextrin to ganaxolone needed to inhibit or prevent precipitation of ganaxolone in the formulation or upon injection depends on the particular type of substituted-β-cyclodextrin used. When CAPTISOL is used a CAPTISOL:ganaxolone ratio of about 52:1 to about 85:1, or about 55:1 to about 70: 1, or about 55:1 to about 65:1 or about 55:1 is required. The substituted β-cyclodextrin may be present in an amount greater than that needed to complex the ganaxolone to aid in ganaxolone solubilization.

In all formulations disclosed herein the ganaxolone and sulfobutyl ether-β-cyclodextrin may be in an inclusion complex, and the inclusion complex may be a 1:1 ganaxolone: sulfobutyl ether-β-cyclodextrin complex.

In certain embodiments the ganaxolone and sulfobutyl ether-β-cyclodextrin inclusion complex provides at least 2.0 mg/ mL ganaxolone (or at least 0.1 mg/ mL, or at least 1.0 mg/ mL, or at least 1.5 mg/ mL), when the amount of ganaxolone provided by the complex is measured at a sulfobutyl ether-β-cyclodextrin concentration of 30% w/v in water. In certain embodiments the ganaxolone concentration is about 0.1 mg/ ml to about 15 mg/ ml, or about 1 mg/ ml to about 10 mg/ ml, or about 1 mg/ ml to about 5 mg/ ml.

In certain embodiments ganaxolone will be present in the aqueous injectable formulation in an amount of from about 0.1 to about 5% by weight, or from about 0.2 to about 2.5%, or from about 0.5 to about 1.5% by weight based on the total injectable formulation weight.

The disclosure also provides injectable substituted β-cyclodextrin ganaxolone formulations containing substituted β-cyclodextrin at a concentration of 5mg/mL, 10mg/ml, 50 mg/ mL, 100 mg/ mL, 150 mg/ mL, 200 mg/ mg/ mL, 250 mg/ mL, 300 mg/ mL, 350 mg/ mL, 400 mg/ mL, 450 mg/ mL, 500 mg/ mL, 550 mg/ mL, 600 mg/ mL, 650 mg/ mL, or 700 mg/ mL so long as the ratio of substituted β-cyclodextrin to ganaxolone is about 52: 1 or greater. All ranges including any two of the foregoing concentrations of substituted β-cyclodextrin as endpoints are also included in the disclosure. For example, the disclosure includes substituted β-cyclodextrin ganaxolone formulations containing from about 5 mg/ mL to about 500 mg/ mL, or about 50 mg/ mL to about 500 mg/ mL, or about 100 mg/ mL to about 400 mg/ mL substituted β-cyclodextrin. The disclosure includes an embodiment in which the substituted β-cyclodextrin ganaxolone formulations contain from about 25 mg/ mL to about 400 mg/ mL sulfobutyl ether-β-cyclodextrin.

Ganaxolone will form a complex with the substituted-β-cyclodextrin which complex may be dissolved in water to form an injectable formulation. However, physical mixtures of ganaxolone and substituted-β-cyclodextrin are within the scope of the disclosure.

Substituted-β-cyclodextrin-ganaxolone formulations of the disclosure may be formed of dry physical mixtures of ganaxolone and substituted-β-cyclodextrin or dry inclusion complexes which are reconstituted upon addition of water to form an aqueous injectable formulation. Alternatively, the aqueous injectable formulation may be lyophilized and later reconstituted with water.

The disclosure includes embodiments in which the ganaxolone-sulfobutyl ether-β-cyclodextrin formulation additionally comprises a buffer, such as an acetate, citrate, tartrate, phosphate, or triethanolamine (TRIS) buffer an acid or base buffer to adjust pH to desired levels. In some embodiments the desired pH is 2.5-11 .0, 3.5 - 9.0, or 5.0 - 8.0, or 6.0 -8.0, or 6.8 - 7.6, or 6.80 -7.10, or about 7.4. Examples of acid buffers useful in the substituted β-cyclodextrin-ganaxolone formulation include oxalic acid, maleic acid, fumaric acid, lactic acid, malic acid, tartaric acid, citric acid,

benzoic acid, acetic acid, methanesulfonic acid, histidine, succinic acid, toluenesulfonic acid, benzenesulfonic acid, ethanesulfonic acid and the like. Acid salts of the above acids may be employed as well. Examples of base buffers useful in the formulation include carbonic acid and bicarbonate systems such as sodium carbonate and sodium bicarbonate, and phosphate buffer systems, such as sodium monohydrogen phosphate and sodium dihydrogen phosphate. In certain embodiments the buffer is a phosphate buffer. In certain embodiments the buffer is phosphate buffered saline. In certain embodiments the buffer is a mixture of monobasic and dibasic phosphate buffers, such as potassium phosphate mono or dibasic phosphate buffers. The concentration of each component of a phosphate buffer system will be from about 5 mM to about 20 mM, about 10 mM to about 200 mM, or from about 20 mM to about 150 mM, or from about 50 mM to about 100 mM.

The disclosure includes embodiments in which the pH of the ganaxolone -sulfobutyl ether-β-cyclodextrin formulation is about 6.9, 7.0, 7.1, 7.2, 7.3, or 7.4.

The formulation may contain electrolytes, such as sodium or potassium. The disclosure includes embodiments in which the formulation is from about 0.5% to about 1.5% sodium chloride (saline).

The formulation may contain tonicity adjusting agents so that it is isotonic with human plasma. Examples of tonicity adjusting agents useful in the formulation include, but are not limited to, dextrose, mannitol, sodium chloride, or glycerin. In certain embodiments the tonicity agent is 0.9 % sodium chloride.

The substituted cyclodextrin-ganaxolone injectable formulation may contain a non-aqueous carrier. Non-aqueous carriers include any pharmaceutically acceptable excipient compatible with ganaxolone and capable of providing the desired pharmacological release profile for the dosage form. Carrier materials include, for example, suspending agents, surfactants, solubilizers, stabilizers, lubricants, wetting agents, diluents, and the like. "Pharmaceutically compatible carrier materials" may comprise, but are not limited to, acacia, gelatin, colloidal silicon dioxide, calcium glycerophosphate, calcium lactate, maltodextrin, glycerin, magnesium silicate, polyvinylpyrrolidone (PVP), cholesterol, cholesterol esters, sodium caseinate, soy lecithin, taurocholic acid, phosphotidylcholine, sodium chloride, tricalcium phosphate, dipotassium phosphate, cellulose and cellulose conjugates, sugars sodium stearoyl lactylate, carrageenan, monoglyceride, diglyceride, pregelatinized starch, and the like.

The substituted β-cyclodextrin-ganaxolone injectable formulation may also contain a non-aqueous diluent such as ethanol, one or more polyol (e.g glycerol, propylene glycol), an oil carrier, or any combination of the foregoing.

The substituted β-cyclodextrin-ganaxolone injectable formulation additionally comprises a preservative.. The preservative may be used to inhibit bacterial growth. Preservatives suitable for parenteral formulations include benzyl alcohol, chlorbutanol, 2-ethoxyethanol, parabens (methyl, ethyl, propyl, butyl, and combinations), benzoic acid, sorbic acid, chlorhexidene, phenol, 3-cresol, thimerosal, and phenylmercurate salts.

The substituted β-cyclodextrin-ganaxolone injectable formulation may optionally include a coating or surfactant to insure desirable solubilization and fluidity of ganaxolone in the substituted β-cyclodextrin, such as CAPTISOL.

Surfactants include compounds such as lecithin (phosphatides); sorbitan trioleate and other sorbitan esters; polyoxyethylene sorbitan fatty acid esters (e.g., the commercially available TWEENS such as polyoxyethylene sorbitan monolaurate (TWEEN 20, also known as Polysorbate 20, CAS Reg. No. 9005-64-5) and polyoxyethylene sorbitan monooleate (TWEEN 80, ICI Speciality Chemicals, also known as Polysorbate 80 (CAS Reg. No. 9005-65-6)); poloxamers (e.g., poloxamer 188 (PLURONIC F68) and poloxamer 338 (PLURONIC F108), which are block copolymers of ethylene oxide and propylene oxide, and poloxamer 407, which is a triblock copolymer of propylene glycol and two blocks of polyethylene glycol); sodium cholesterol sulfate or other cholesterol salts; and bile salts, such as sodium deoxycholate, sodium cholate, sodium glycbolate, salts of deoxycholic acid, salts of glycholic acid, salts of chenodeoxycholic acid, and salts of lithocholic acid.

In an embodiment the disclosure includes a substituted β-cyclodextrin-ganaxolone injectable formulation containing (1) ganaxolone, from 2 to about 8 mg/mL, (2) CAPTISOL, from about 100 to about 400 mg/ mL, (3) phosphate buffer to adjust pH to from about 7.0 to about 7.5, and (4) water. The β -cyclodextrin-ganaxolone injectable formulation may be aseptically filtered, for example, through a 0.2μm membrane filter. The β-cyclodextrin-ganaxolone injectable formulation may be autoclaved or lyophilized for storage and reconstitution.

The disclosure includes any β -cyclodextrin-ganaxolone injectable formulation as described in this disclosure that also meet the following requirements. Any of the following requirements can be combined so long as a stable formulation results. In any of the disclosed injectable β -cyclodextrin-ganaxolone injectable formulations, the ganaxolone and the sulfobutyl ether-β-cyclodextrin are in the form of an inclusion complex.

(a) The β -cyclodextrin-ganaxolone injectable formulation includes a surfactant and the surfactant is a sorbitan ester, a polyoxyethylene sorbitan fatty acid ester, a poloxamer, a cholesterol salt, or a bile salt.
(b) The β -cyclodextrin-ganaxolone injectable formulation is about 0.05 to about 15 weight percent surfactants.
(c) The β -cyclodextrin-ganaxolone injectable formulation includes a surfactant wherein the surfactant is polysorbate 80.
(d) The β -cyclodextrin-ganaxolone injectable formulation includes a buffer.

(e) The β -cyclodextrin-ganaxolone injectable formulation includes a buffer having a pH of about 6.8 to about 7.6.

(f) The β -cyclodextrin-ganaxolone injectable formulation includes a phosphate buffer. In certain embodiments the phosphate buffer is phosphate buffered saline. The buffer is a combination of a monobasic phosphate buffer and a dibasic phosphate buffer, wherein the concentration of each phosphate buffer is 2 mM to 50 mM. In certain embodiments the phosphate buffer is a combination of a monobasic phosphate buffer and a dibasic phosphate buffer, wherein the concentration of each phosphate buffer is 2 mM to 50 mM.

(g) The β -cyclodextrin-ganaxolone injectable formulation includes a concentration of ganaxolone that is 2 mg/ ml to 8 mg/ ml; the w/w ratio of sulfobutyl ether-β-cyclodextrin to ganaxolone is within the range from about 52:1 to about 90:1, or about 52:1 to about 80:1, or about 55:1 to about 70:1; or at least 55:1; the formulation contains a buffer and has a pH of 6.7 to 7.3; and the formulation contains from 0.5 to 15 weight percent surfactant, or about 1 to about 10 weight percent surfactant; or about 10 weight percent surfuactant.

(h) The sulfobutyl ether β -cyclodextrin-ganaxolone injectable formulation includes a concentration of ganaxolone that is from 1 mg/ ml to 5 mg/ ml; the weight percent of sulfobutyl ether-β-cyclodextrin 20% to 40%; or 25% to 35%, or about 30% sulfobutyl ether β -cyclodextrin and the formulation contains from 5% to 20%; 5% to 15%; or about 10 % (weight percent) of at least one of the following: a surfactant, ethanol, glycerin or propylene glycol. In certain embodiments this formulation may contain a surfactant chosen from lecithin (phosphatides); sorbitan trioleate and other sorbitan esters; polyoxyethylene sorbitan fatty acid esters (e.g., the commercially available TWEENS such as polyoxyethylene sorbitan monolaurate and polyoxyethylene sorbitan monooleate; poloxamers (e.g., poloxamer 188 (PLURONIC F68) and poloxamer 338 (PLURONIC F108), which are block copolymers of ethylene oxide and propylene oxide, and poloxamer 407, which is a triblock copolymer of propylene glycol and two blocks of polyethylene glycol); sodium cholesterol sulfate or other cholesterol salts; and bile salts

(i) The β-cyclodcxtnn-ganaxolone injectable formulation includes a preservative. In certain embodiments the preservative is benzyl alcohol, chlorbutanol, 2-ethoxyethanol, parabens (including methyl, ethyl, propyl, butyl, and combinations), benzoic acid, sorbic acid, chlorhexidene, phenol, 3-cresol, thimerosal, or a phenylmercurate salt.

(j) The sulfobutyl ether-β-cyclodextrin are in the form of an inclusion complex, wherein the inclusion complex provides at least 2.0 mg/ mL ganaxolone, when the amount of ganaxolone provided by the complex is measured at a sulfobutyl ether-β-cyclodextrin concentration of about 30% w/v in water.

(k) The β-cyclodextrin-ganaxolone injectable formulation includes ethanol, e.g. 1 to 20 percent (volume/ volume), 5 to 15 percent (v/v), or about 10 percent ethanol (v/v).

(l) The β-cyclodextrin-ganaxolone injectable formulation includes a wetting agent. In certain embodiments the solubilizing agent is glycerin or propylene glycol, e.g. 1 to 20 percent (volume/ volume), 5 to 15 percent (v/v), or about 10 percent (v/v).

LYOPHILIZED BETA-CYCLODEXTRIN-GANAXOLONE FORMULATIONS

The disclosure includes lyophilized forms of all formulations disclosed herein.

The disclosure provides injectable substituted β-cyclodextrin ganaxolone formulations containing an inclusion complex of a substituted-β-cyclodextrin, such as CAPTISOL, and ganaxolone, and a pharmaceutically acceptable carrier. In certain embodiments the substituted β-cyclodextrin ganaxolone formulation is a lyophilized form that is dissolved in water or an aqueous solution prior to administration. The disclosure includes embodiments in which the lyophilized ganaxolone formulation can be reconstituted in water to provide a clear solution.The lyophilized form may additionally include a bulking agent, a stabilizer, a buffer (or pH adjuster), a tonicity adjuster, or a combination of any of the foregoing. In certain embodiments the lyophilized formulation includes one or more of a surfactant, a buffer, and a preservative.

Bulking agents are useful for lyophilized formulation in which a low concentration of the active ingredient, or in the present case, in which a low concentration of the inclusion complex, is present. Bulking agents include mannitol, lactose, sucrose, trehalose, sorbitol, glucose, rafinose, glycine, histidine, polyethylene glycol (PEG), and polyvinyl pyrrolidone (PVP).

The removal of the hydration shell from an active agent during lyophilization can be destabilizing. In certain embodiments the lyophilized form contains a stabilizer. Stabilizers include agents which maintain a desirable attribute of the formulation over a time interval including but not limited to mechanical, chemical and temperature stressing that can be tested in a laboratory setting. Such attributes include stable particle size or homogeneity resulting in concentrations consistent with the labeled potency and maintaining purity.

Suitable stabilizers include sugars such as sucrose, trehalose, glucose, and lactose.

METHODS OF TREATMENT

The disclosure includes methods of treating status epilepticus, refractory status epilepticus, super-refractory status epilepticus, PCDH19 female pediatric epilepsy, and other seizure disorders comprising administering an effective

amount of the substituted β-cyclodextrin-ganaxolone injectable formulation to a patient suffering from any of these seizure disorders.

Seizure disorders that may be treated with the substituted β-cyclodextrin-ganaxolone injectable formulation include status epilepticus, e.g., convulsive status epilepticus, e.g., early status epilepticus, established status epilepticus, refractory status epilepticus, super-refractory status epilepticus, e.g., super-refractory generalized status epilepticus; non-convulsive status epilepticus, e.g., generalized status epilepticus, complex partial status epilepticus; a seizure, e.g., acute repetitive seizures, cluster seizures, infantile spasms, Lennox-Gastaut syndrome, West syndrome, PCDH19 female pediatric epilepsy, and catamenial epilepsy.

The substituted β-cyclodextrin-ganaxolone injectable formulation may also be used to treat provoked seizures such as seizures resulting from low blood sugar, electrolyte imbalance, high fever, brain infection (such as brain infections due to encephalitis, malaria, meningitis, toxoplasmosis, or amoebic infection), adverse reaction to prescription drugs, or alcohol or drug overdose.

The disclosure also includes methods of using substituted β-cyclodextrin-ganaxolone injectable formulation to treat traumatic brain injury and stroke comprising administering an effective amount of the formulation to a patient suffering from recent traumatic brain injury or a recent stroke.

The disclosure further includes methods of treating seizures arising from neurodegenerative disorders. Such neurodegenerative disorders include Parkinson's disease, Alzheimer's Disease, Amyotrophic Lateral Sclerosis, and Huntington's Disease. The disclosure includes methods of treating seizure arising from inflammatory disorders, such as multiple sclerosis. The disclosure includes methods of treating seizure disorders arising from lysosomal storage disorders including Neimann-Pick-C, Tay Sachs, Batten, Sandhoff, and Gaucher disease.

Methods of treatment include treating a patient suffering from seizures, traumatic brain injury, or stroke by administering a single injection (bolus dose) of a substituted β-cyclodextrin-ganaxolone injectable formulation. The single injection may be administered intramuscularly or intravenously. The dose of the single injection may be from about 1 mg/ kg to about 20 mg/ kg, from about 2 mg/ kg to about 15 mg/ kg, from about 2 mg/ kg to about 10 mg/ kg, or about 2 mg/ kg to about 8 mg/ kg. Methods of treatment also include administering multiple injections of the β-cyclodextrin-ganaxolone injectable formulation over a period of 1 to 10 days. The injections may be given at intervals of 1 to 24 hours. Dosing schedules in which the substituted β-cyclodextrin-ganaxolone injectable formulation is injected every 1 hr, 2 hrs, 4 hrs, 6 hrs, 8 hrs, 12 hrs, or 24 hours are included herein. Dosing schedules in which the substituted β-cyclodextrin-ganaxolone injectable formulation is injected for 1, 2, 3, 4, 5, 6, 7, 8, 9, or 10 days are included herein.

Methods of treatment include treating a patient suffering from seizures, traumatic brain injury, or stroke by administering one or more bolus doses over a period of 1 to 10 days as described in the preceding paragraph of a substituted β-cyclodextrin-ganaxolone injectable formulation followed by an intravenous infusion of the substituted β-cyclodextrin-ganaxolone injectable formulation. In certain embodiments the bolus dose is administered over a period of about 1 to about 30, about 1 to about 15, about 1 to about 10, or about 1 to about 5, or about 5 minutes followed by commencement of the intravenous infusion within 1, 2, 3, 4, or 5 hours.

In some embodiments, substituted β-cyclodextrin-ganaxolone injectable formulation is administered as an intravenous infusion dose, either without or without a previous bolus dose for 1, 2, 3, 4, 5, 6, 7, 8, 9, or 10 consecutive days. The infusion dose may be administered at a rate of 1, 2, 3, 4, 5, 6, 7, 8, 9, or 10 mg/ kg/ hr or in a range of about 1 mg/ kg/ hr to about 10 mg/ kg/ hr or 2 mg/ kg/ hr to about 8 mg/ kg / hrs.

In some embodiments the infusion dose (whether administered with or without the bolus dose) is followed by a first step down dosage, and optionally a second step down dosage, an optionally a third step down infusion dosage. In some embodiments, the first step dose is 95%, 90%, 85%, 80%,75%,70%, 65%, 60%, 55%, 50%,45%, 40%, 35%, 30%, 25%, 20%, 15%, 10%, or 5% of the infusion dose. In some embodiments, the first step dose is between 95-50%, 75-50%, 85-50%, 90-50%, 80-50%, or 75-100% of the infusion dose. In an embodiment, the first step dose is 75% of the infusion dose. In some embodiments, the second step dose is 95%, 90%, 85%, 80%, 75%, 70%, 65%, 60%,55%,50%,45%,40%, 35%, 30%, 25%, 20%, 15%, 10%, or 5% of the first step down dose. In some embodiments, the second step dose is between 95-30%, 75-30%, 85-30%, 60-30%, 70-30%, 50-30%, or 50-40% of the first step down dose. In an embodiment, the second step dose is 50% of the infusion dose. In some embodiments, the third step dose is 95%, 90%, 85%, 80%, 75%, 70%, 65%, 60%, 55%, 50%, 45%, 40%, 35%, 30%, 25%, 20%, 15%, 10%, or 5% of the second infusion dose. In some embodiments, the third step dose is between 50-5%, 40-5%, 30-5%, 25-5%, 25-10%, 25-20%, or 25-40% of the second step down dose. In an embodiment, the third step down dose is 25% of the infusion dose.

The disclosure includes methods of treating a seizure disorder wherein the seizure disorder is status epilepticus, refractory status epilepticus, super refractory status epilepticus, or PCDH19 female pediatric epilepsy comprising administering an effective amount of the ganaxolone/ sulfobutyl ether-β-cyclodextrin formulation to a patient.

The disclosure includes methods of treating a seizure disorder, stroke, or traumatic brain injury, comprising administering an effective amount of the ganaxolone/ sulfobutyl ether-β-cyclodextrin formulation to a patient wherein the amount of ganaxolone administered is from about 1 mg/kg to about 200 mg/kg.

In certain embodiments the ganaxolone/ sulfobutyl ether-β-cyclodextrin formulation is administered intramuscularly or intravenously.

The disclosure includes embodiments in which the ganaxolone/ sulfobutyl ether-β-cyclodextrin formulation is administered as a single bolus dose of the ganaxolone formulation to the patient. In certain embodiments the single bolus dose provides a sufficient amount of ganaxolone to provide a plasma $C_{max}$ of ganaxolone of about 100 ng/ mL to about 1000 ng/ mL in the patient.

The disclosure includes embodiments in which the ganaxolone/ sulfobutyl ether-β-cyclodextrin formulation is administered as a bolus dose and the bolus dose provides a sufficient amount of ganaxolone to provide a plasma $C_{max}$ of ganaxolone of about 600 ng/ mL to about 900 ng/ mL in the patient.

The disclosure includes embodiments in which the ganaxolone/ sulfobutyl ether-β-cyclodextrin formulation is administered as a bolus dose and the bolus dose is administered in less than 10 minutes and the $C_{max}$ occurs within 1 hour of completion of administration.

The disclosure includes embodiments in which the ganaxolone/ sulfobutyl ether-β-cyclodextrin formulation is administered as a single bolus dose and the single bolus dose comprises from about 1 mg/kg to about 20 mg/kg ganaxolone. Or, optionally the single bolus dose comprises from about 2 mg/kg to about 15 mg/kg ganaxolone, or about 4 mg/kg to about 10 mg/kg ganaxolone, or from about 1 mg/kg to about 30 mg/kg ganaxolone.

The disclosure includes embodiments in which multiple bolus doses of the ganaxolone/ sulfobutyl ether-β-cyclodextrin formulation are administered to the patient. In certain embodiments the multiple bolus doses are given over 1 to 10 days at intervals of 1 to 24 hours. In certain embodiments each bolus dose provides a sufficient amount of ganaxolone to produce a plasma $C_{max}$ of ganaxolone of about 100 ng/ mL to about 1000 ng/ mL in the patient. In certain embodiments the interval between bolus doses is from about 10 to about 24 hours and once an initial $C_{max}$ is reached the plasma concentration of ganaxolone is not below 100 ng/ mL at any time between bolus doses. In certain embodiments the interval between bolus doses is 20 to 24 hours and once an initial $C_{max}$ is reached and the concentration of ganaxolone in the patient's plasma does not fall below 25% of the initial $C_{max}$. In certain embodiment each bolus dose comprises about 1 mg/kg to about 20 mg/kg ganaxolone. Or, optionally the single bolus dose comprises from about 2 mg/kg to about 15 mg/kg ganaxolone, or about 4 mg/kg to about 10 mg/kg ganaxolone, or from about 1 mg/kg to about 30 mg/kg ganaxolone.

In certain embodiments the method comprises administering an infusion of the ganaxolone/ sulfobutyl ether-β-cyclodextrin formulation to the patient, with or without an initial bolus dose. In certain embodiments the infusion is administered for 1 to 10 consecutive days at a rate of 1 to 10 mg/kg/hr without an initial bolus dose.

In certain embodiments the method comprises administering an initial bolus dose of the ganaxolone/ sulfobutyl ether-β-cyclodextrin formulation comprising from about 1 mg/kg to about 20 mg/kg ganaxolone, followed within 24 hours by administration of an infusion of the ganaxolone formulation for 1 to 10 consecutive days at a rate of 1 to 10 mg/kg/hr.

In certain embodiments the method comprises administering an initial bolus dose of the ganaxolone/ sulfobutyl ether-β-cyclodextrin formulation followed by an infusion dose, wherein the initial bolus dose provides a sufficient amount of ganaxolone to provide an initial plasma $C_{max}$ of ganaxolone of about 100 ng/ mL to about 1000 ng/ mL in the patient and the concentration of ganaxolone in the patient's plasma does not fall below 25% of the initial $C_{max}$ until after the subsequent infusion dosing is concluded.

In certain embodiments the method comprises administering an initial bolus dose of the ganaxolone/ sulfobutyl ether-β-cyclodextrin formulation, wherein the initial bolus dose provides a sufficient amount of ganaxolone to provide an initial plasma $C_{max}$ of ganaxolone of about 100 ng/ mL to about 1000 ng/ mL in the patient, the patient is then administered an infusion of the ganaxolone formulation at a constant dose sufficient to provide a concentration of ganaxolone in the patient's plasma of at least 40 % of $C_{max}$, followed by an infusion of ganaxolone at a gradually reducing dose so that the concentration of ganaxolone in the patient's plasma is less than 20% of $C_{max}$ when the infusion is concluded.

## COMBINATION TREATMENT

The disclosure includes embodiments in which ganaxolone is the only active agent and embodiments in which ganaxolone is administered in combination with one or more additional active agents. When used in combination with an additional active agent ganaxolone and the additional active agent may be combined in the same formulation or may be administered separately. Ganaxolone may be administered while the additional active agent is being administered (concurrent administration) or may be administered before or after the additional active agent is administered (sequential administration).

The disclosure includes embodiments in which the additional active agent is an anti-convulsant. Anticonvulsants include $GABA_A$ receptor modulators, sodium channel blocker, GAT-1 GABA transporter modulators, GABA transaminase modulators, voltage-gated calcium channel blockers, and peroxisome proliferator-activated alpha modulators.

The disclosure includes embodiments in which the patient is given an anesthetic or sedative in combination with ganaxolone. The anesthetic or sedative may be administered at a concentration sufficient to cause the patient to

lose consciousness, such as a concentration sufficient to medically induce coma or a concentration effective to induce general anesthesia. Or the anesthetic or sedative may be given at a lower dose effective for sedation, but not sufficient to induce a loss of consciousness.

A medically induced coma occurs when a patient is administered a dose of an anesthetic, such as propofol, pentobarbital or thiopental, to cause a temporary coma or a deep state of unconsciousness. General anesthesia is a treatment with certain medications to cause unconsciousness sufficient to be unaware of pain during surgery. Drugs used for medically induced coma or general anesthesia include inhalational anesthetics and intravenous anesthetics which include barbiturate and non-barbiturate anesthetics.

Inhalational anesthetics include desflurane, enflurane, ethyl chloride, halothane, isoflurane, methoxyflurane, sevoflurane, and trichloroethylene.

Intravenous, non-barbiturate anesthetics include atracurium, cisatracurium, etodimidate, ketamine, propofol, and rocuronium,

Barbiturates include amobarbital, methohexital, pentobarbital, phenobarbital, secobarbital, thiamylal, and thiopental.

Benzodiazepines are used both as anticonvulsants and anesthetics. Benzodiazepines useful as anaesthetics include diazepam, flunitrazepam, lorazepam, and midazolam.

The disclosure includes administering propofol to induce anesthesia in combination with ganaxolone. Propofol is administered at a dose range or dosage range of 0.5- 50mg/kg. Anesthesia is induced with an initial bolus of 10 - 50 mg/ kg followed by additional intermittent boluses or 10 - 50 mg/ kg to maintain anesthesia. Anesthesia may also be maintained by an infusion of 3 - 18 mg/kg/min propofol.

The disclosure includes administering pentobarbital sodium by intravenous or intramuscular injection to induce anesthesia in combination with ganaxolone. Pentobarbital may be administered to adults as a single 100-500 mg, or 100-200 mg intramuscular or intravenous injection, or to pediatric patients as a single 2 to 6 mg/ kg IM or IV injection. Pentobarbital may be administered at a high dose to induce coma in a status epilepticus patient and ganaxolone may then be given in combination with the pentobarbital to treat refractory seizures. Pentobarbital doses used to induce coma include, a loading dose of 5 to 15 mg/ kg or 10 to 35 mg/ kg, given over 1-2 hours followed by a maintenance dose of 1 mg/ kg/ hr to 5 mg/ kg/ hr for 12 to 48 hours and tapering by 0.25 to 0.5 mg/ kg/ hr every 12 hours once seizures have stopped.

The disclosure includes administering thiopental sodium in combination with ganaxolone. Thiopental can be administered as a 3 to 5 mg/ kg bolus followed by additional boluses of 1 to 2 mg/kg every 3 to 5 minutes until seizures have stopped, to a maximum total dose of 10 mg/kg. After the 10 mg/ kg maximum bolus dose of thiopental has been reached, thiopental can be infused at 3 to 5 mg/ kg/ hr.

The disclosure includes administering midazolam in combination with ganaxolone. Midazolam can be administered as a 0.5 mg/ kg to 5 mg/ kg loading dose, followed by a 1 to 5 microgram/ kg/ hour infusion.

In each embodiment in which an additional active agent is administered to induce coma, anesthesia, or sedation, ganaxolone is administered as an injectable substituted-β-cyclodextrin ganaxolone formulation and is administered simultaneously or sequentially with the additional active agent and is administered according to any of the dosing schedules set forth herein for ganaxolone administration.

The injectable substituted-β-cyclodextrin ganaxolone formulation of this disclosure may be administered with another anticonvulsant agent. Anticonvulsants include a number of drug classes and overlap to a certain extent with the coma-inducing, anesthetic, and sedative drugs that may be used in combination with ganaxolone. Anticonvulsants that may be used in combination with the injectable substituted-β-cyclodextrin ganaxolone formulation of this disclosure include aldehydes, such as paraldehyde; aromatic allylic alcohols, such as stiripentol; barbiturates, including those listed above, as well as methylphenobarbital and barbexaclone; benzodiazepines include alprazolam, bretazenil, bromazepam, brotizolam, chloridazepoxide, cinolazepam, clonazepam, chorazepate, clopazam, clotiazepam, cloxazolam, delorazepam, diazepam, estazolam, etizolam, ethyl loflazepate, flunitrazepam, flurazepam, flutoprazepam, halazepam, ketazolam, loprazolam, lorazepam, lormetazepam, medazepam, midazolam, nimetazepam, nitrazepam, nordazepam, oxazepam, phenenazepam, pinazepam, prazepam, premazepam, pyrazolam, quazepam, temazepam, tatrazepam, and triazolam; bromides, such as as potassium bromide; carboxamides, such carbamazepine, oxcarbazepine, and eslicarbazepine acetate; fatty acids, such as valproic acid, sodium valproate and divalproex sodium; fructose derivatives, such as toprimate; GABA analogs such as gabapentin and pregabalin, hydantoins, such as ethotoin, phenytoin, mephenytoin, and fosphenytoin; other neurosteroids, such as allopregnanolone, oxasolidinediones, such as paramethadione, trimethadione, and ethadione, propionates such as beclamide; pyrimidinediones such as primidone, pyrrolidines such as brivaracetam, levetiracetam, and seletracetam, succmimides, such as ethosuximide, pensuximide, and mesuximide; sulfonamides such as acetazoloamide, sultiame, methazolamide, and zonisamide; triazines such as lamotrigine, ureas such as pheneturide and phenacemide; NMDA antagonists, such as felbamate, and valproylamides such as valpromide and valnoctamide; and perampanel. In certain embodiments the disclosure includes the use of and injectable sulfobutyl ether β-cyclodextrin - ganaxolone formulation in combination with diazepam. The diazepam may be combined in the

injectable sulfobutyl ether β-cyclodextrin - ganaxolone or may be administered in separate dosage forms. For example, the disclosure includes an injectable sulfobutyl ether β-cyclodextrin - ganaxolone formulation comprisition 1 mg/ ml to 10 mg/ ml ganaxolone, 25% to 35% (weight percent) sulfobutyl ether β-cyclodextrin and 1 mg/ ml to 40 mg/ml diazepam, or from about 2 mg/ ml to about 20 mg/ ml diazepam.

EXAMPLES

EXAMPLE 1. PREPARATION OF INJECTABLE GANAXOLONE FORMULATION

Ganaxolone solubility in aqueous CAPTISOL solution was first determined by constructing a phase solubility diagram. Excess ganaxolone was shaken in aqueous CAPTISOL solutions of known concentrations for 42 hours to reach equilibrium. The ganaxolone solution was filtered into HPLC vials through 0.45 μm syringe filters. The filtrates were assayed for ganaxolone concentration by HPLC. The results are summarized in Table 1. Moles of ganaxolone in solution against moles of CAPTISOL added were plotted. Ganaxolone solubility in water was found to increase linearly with the addition of CAPTISOL indicating the formation of 1:1 complex between ganaxolone and CAPTISOL. A plot of weight (mg) of ganaxolone in solution against weight (mg) of CAPTISOL added (FIGURE 1), shows the weight: weight ratio of CAPTISOL to ganaxolone required for ganaxolone solubilization at equilibrium to be approximately 52:1.

TABLE 1

| No. | CAPTISOL conc. (mg/ mL) | CAPTISOL conc. (M) | Ganaxolone conc. by HPLC (mg /mL) | Ganaxolone conc. (M) |
|---|---|---|---|---|
| 1 | 400 | 0.185 | 7.68 | 0.0231 |
| 2 | 200 | 0.0925 | 3.63 | 0.0109 |
| 3 | 100 | 0.0462 | 1.86 | 0.0056 |
| 4 | 50 | 0.0231 | 0.88 | 0.0027 |
| 5 | 25 | 0.0116 | 0.43 | 0.0013 |
| 6 | 0 | 0 | Not detectable | 0 |

To prepare injectable solutions, excess ganaxolone is added to an aqueous 400 mg/ mL CAPTISOL solution. The solution is shaken at least overnight and filtered through a 0.45 micron filter. Ganaxolone concentration of the filtered solution is determined by HPLC. The ganaxolone/ CAPTISOL solution (7.68 mg/ mL in 400 mg/ mL aqueous CAPTISOL) is diluted in saline to obtain 3.84 mg/ mL, 0.77 mg/ mL and 0.39 mg/ mL ganaxolone solutions in 0.9% saline. All solutions were clear and free from any visible precipitation. The ganaxolone solutions remained free of any visible precipitation after freezing and thawing.

EXAMPLE 2. PREPARATION OF INJECTABLE GANAXOLONE-CAPTISOL SOLUTION (5 MG/ML)

Ganaxolone (0.50 g) was first mixed manually using a spatula with a small amount (approximately 20 mL) of 30% w/v CAPTISOL solution in sterile water for injection to form a uniform paste. Additional amount (approximately 40 mL) of 30% w/v CAPTISOL solution was then added to obtain a slurry. The suspension was stirred using a magnetic stir bar for 20 min. It was sonicated using a probe sonicator for 2h. While sonicating, an additional 30% w/v CAPTISOL solution was added until total amount of the CAPTISOL solution reached 99.58 mL. The stirred formulation was then heated at 68.5 °C for about 2.5 hours to obtain a solution. Heat was removed and the solution was stirred at room temperature for approximately 2h. Volume lost due to evaporation was replenished with water. The clear solution was sterile filtered through 0.2 μm Nylon membrane filter.

EXAMPLE 3. PREPARATION OF A LYOPHILIZED GANAXOLONE-CAPTISOL POWDER

A ganaxolone-CAPTISOL solution was prepared by dissolving 42.6 mg of ganaxolone in 6.6 mL of 40% w/v CAPTISOL and stirred 1 hr (a small amount of undissolved ganaxolone was removed by filtration over 0.45 μm syringe filter in order to obtain a clear solution). This solution was frozen in a dry ice/acetone bath and lyophilized for 2 days to obtain 2.859 g of a free flowing white powder. The ganaxolone concentration of the lyophilized powder was assayed by HPLC to be 1.26% by weight which is slightly lower than theoretical (1.49% by weight). The lyophilized powder was reconstituted in water to obtain clear solution.

EXAMPLE 4. BUFFERED GANAXOLONE-CAPTISOL SOLUTIONS

Monobasic potassium phosphate (19.6 mg) and dibasic sodium phosphate heptahydrate (93.3 mg) were added to 3 mg/mL ganaxolone solution in 30% Captisol (20 mL) with an initial pH 4.53. The mixture was sonicated for 1 min to obtain a clear solution having pH 6.95. About 10 mL of this solution was maintained at 80 °C with magnetic stirring alongside the unbuffered control in closed glass vials. The remaining 10 mL of the sample was kept at 5 °C as control. Aliquots were taken at 67 hours and 5 days. The samples were analyzed by HPLC and results are shown in Table 2.

Table 1. Stability of unbuffered and phosphate buffered ganaxolone/Captisol solution after 5 days at 80 °C

| TABLE 2. Stability of Buffered Ganaxolone Solutions | | | | | | |
|---|---|---|---|---|---|---|
| | 5 days at 5 °C | | 67 h at 80 °C | | 5 days at 80 °C | |
| Degradation product | Unbuffered | Phosphate Buffered PH 6.95 | Unbuffered | Phosphate buffered pH 6.95 | Unbuffered | Phosphate Buffered PH 6.95 |
| 3-Epimer of ganaxolone | ND | ND | 0.64% | ND | 1.89% | 0.03% |
| 17-Epimer of ganaxolone | ND | ND | 0.13% | 0.07% | 0.39% | 0.13% |
| ND, Not detected | | | | | | |

EXAMPLE 5. INJECTABLE GANAXOLONE- 30% CAPTISOL SOLUTION CONTAINING POLYSORBATE 80

500 μl of 10% aqueous Polysorbate 80 solution in a 20 ml scintillation vial were combined with powdered ganaxolone (50 mg). The mixture was stirred to wet the ganaxolone. Solid Captisol (3.2 g), sufficient to form a 30% Captisol solution, was then added to the vial and the vial contents were mixed. Deionized water (8.0 g) was then added and the mixture was vigorously stirred at room temperature overnight to obtain a hazy solution. An aliquot was filtered through 0.2 μm syringe filter and ganaxolone concentration was assayed by HPLC to be 4.28 mg/ml.
The following injectable ganaxolone-30% Captisol solutions were prepared by the methods of this example.

(a) Ganaxolone-30% Captisol solution containing ethanol.

Ganaxolone was vigorously stirred in 30% Captisol in the presence of 10% v/v ethanol overnight to obtain a 3.16 mg/ ml ganaxolone solution.
(b) Ganaxolone-30% Captisol solution containing glycerin
Ganaxolone was vigorously stirred in 30% Captisol in the presence of 10% v/v glycerin overnight to obtain a 3.45 mg/ml ganaxolone solution.
(c) Ganaxolone-30% Captisol solution containing propylene glycol
Ganaxolone was vigorously stirred in 30% Captisol in the presence of 10% v/v propylene glycol overnight to obtain a 2.53 mg/ ml ganaxolone solution.

EXAMPLE 6. PREPARATION OF GANAXOLONE/30% CAPTISOL SOLUTION BY PRIOR DRY MIXING OF GANAXOLONE AND CAPTISOL

Powdered ganaxolone (125 mg) was charged into 100 ml beaker. Captisol powder (7.9 g) was then added to the beaker. The mixture was mixed by stirring with a magnetic stir bar for 5 min. Deionized water (20.1 g) was weighed into a plastic cup. About half of the water was added into the beaker and the contents were vigorously stirred for 30 minutes to obtain a homogeneous mixture. The remaining water was added and the beaker was covered with paraffin film. The contents were stirred vigorously at room temperature overnight to obtain a 4.61 mg/ ml ganaxolone solution. After 90 hours of stirring, the ganaxolone concentration remained unchanged.

EXAMPLE 7. PLASMA LEVELS OF GANAXOLONE IN RATS INJECTED AND INFUSED WITH GANAXOLONE-CAPTISOL SOLUTION

Three groups of male and female Crl:CD(SD) rats, 100-300 g (Charles River Laboratories, Raleigh, North

Carolina), were given an 8 to 24-hour continuous intravenous infusion dose of ganaxolone-CAPTISOL solution in sterile water for injection (ganaxolone:CAPTISOL = 1:60, w/w). The first group was infused at a rate of 1 mg/kg/hr. The second group was infused at a rate of 2.5 mg/kg/hr. For the third group, the male rats were infused at a rate of 3.5 mg/kg/hr and the females were infused at 1.75 mg/kg/hr. The plasma concentration over time profiles of ganaxolone are shown in FIGURE 2.

EXAMPLE 8. GANAXOLONE EFFECT ON PILOCARPINE-INDUCED STATUS EPILEPTICUS IN RATS

This study was designed to determine the ability of a CAPTISOL-ganaxolone (GNX) formulation administered intravenously (IV) at 12 and 15 mg/kg to inhibit pilocarpine induced status epilepticus (SE). Allopregnanolone (Allo) (15 mg/kg; IV) was also evaluated for comparison. In this study, test drugs were administered either 15 or 60 minutes after onset of SE and compared to vehicle (30% Captisol).

Cortical EEG activity was recorded in rats before and after administration of pilocarpine to induce SE. Animals were pre-treated with LiCl to enhance and improve reliability of seizure induction and scopolamine to reduce peripheral cholinergic side effects of pilocarpine . EEG activity was recorded for 5 h after SE onset, and anticonvulsant activity was assessed by determining changes in EEG power over frequency ranges from 0.3 to 96 Hz.

Test Compounds

The compounds listed in TABLE 3 are used in the pilocarpine induced status epilepticus study.

TABLE 3

| Compound | Source | Formulation |
|---|---|---|
| Lithium Chloride | Sigma #L4408 | 25.4 mg/ mL in sterile water |
| Scopolamine methyl bromide | Sigma #S8502 | 1 mg/ mL in phosphate buffered saline (PBS) |
| Pilocarpine | Sigma #P6503 | 10 mg/ mL in PBS |
| Ganaxolone | Marinus | 2.5 mg/ mL in 30% CAPTISOL/ sterile water, clear solution. pH ~5 |
| Allopregnanolone | Marinus | 2.5 mg/ mL in 30% CAPTISOL/ sterile water, clear solution. pH ~5 |

Animals

Male Sprague-Dawley rats were used for the studies. Rats were surgically implanted with EEG electrodes and jugular vein catheters. The mean weight at the time of recording was 321 ± 3 g (269 - 351 g).

EEG electrodes were surgically implanted in anesthetized rats. Animals were also treated with an anti-inflammatory anaelgesic (RIMADYL, (carprofen)) prior to surgery and a subcutaneous local anesthetic. Stainless steel screw electrodes chronically were implanted in the skull (0-80 x ¼", Plastics-One, Roanoke, VA) such that the ends of the screws were flush with the inner skull surface. One electrode was located 3.0 mm anterior to bregma and 2 mm to the left of midline, and the second 4.0 mm posterior to bregma and 2.5 mm to the right of midline. The skull surface around the electrodes was sealed with super glue and dental acrylic, and the EEG electrode lead wires were inserted into a plastic pedestal mounted on the skull using dental acrylic. Wound edges were treated with triple antibiotic cream (bacitracin zinc, neomycin sulfate, polymyxin B sulfate; Walgreens). Following surgery, animals were administered antibiotic (ampicillin, 50 mg/kg IP at 0.4 mL/kg) and also received a chewable dose (~10 mg) of Rimadyl following surgery. The animals were allowed to recover from EEG surgery for 1 week.

Rats were implanted with jugular-vein catheters (JVC) one week following EEG surgery. Rats were administered an anti-inflammatory / analgesic and then anesthetized and placed in a supine position. An incision was made in the skin on the right ventrolateral aspect of the neck to expose the external jugular vein which was then dissected free of surrounding fascia. A ligature was tied around the vein anteriorly to occlude blood flow returning to the heart. A second ligature was loosely tied around the vein posteriorly to create tension on the vessel during venotomy and catheter insertion. After incising the vein, a PE catheter (3Fr) was inserted into the vein and advanced approximately 30 mm towards the heart, positioning the tip at the junction of the precava and right atrium. After confirming patency of the catheter by blood withdrawal, the posterior ligature was tied off, and the catheter flushed with a "heparin-lock" solution (5 units heparin/ml saline) and plugged with a sterilized stainless steel pin. The catheter was then exteriorized by tunneling through the subcutaneous tissue to exit posterior to the head between the shoulder blades. Lastly, after suturing the

catheter to the skin, all wounds were closed using sutures or wound clips. Immediately following surgery, the animals were awakened to assess catheter patency. Animals also received a chewable dose (~10 mg) of Rimadyl following surgery to minimize pain and inflammation.

Animals were allowed a 1-week recovery time following JVC surgery. Jugular vein catheters were flushed daily with a 0.1 mL of heparin solution (5 units/mL) to maintain patency. On the day of surgery, the flushing solution contained ampicillin sulbactam (50 mg/kg). If any catheters became difficult to flush, the animal number and date was recorded so that the animal could be excluded or assigned to a vehicle group if possible.

EEG Recording Procedure

For EEG recording, each rat was dosed with LiCl and placed into a recording container (30 x 30 x 30 cm with a wire-mesh grid top) the evening prior to recording. Animals were not fasted, and had *ad libitum* access to food and water prior to scopolamine administration, at which time food was removed. The recording container was located inside a sound attenuation cabinet that contained a ventilation fan, a ceiling light, and a video camera.

Cortical EEG signals were fed via a cable attached to a commutator (Plastics-One, Roanoke, VA), then to an amplifier (A-M Systems model 1700; 1000x gain), band pass filtered (0.3 - 1000 Hz), and finally digitized at 512 samples per second using ICELUS acquisition / sleep scoring software (M. Opp, U. Michigan) operating under National Instruments (Austin, TX) data acquisition software (Labview 5.1) and hardware (PCI-MIO-16E-4).

Drug Treatment Groups

TABLE 4 presents the drug treatment group used in this study. All injections were via jugular vein catheter except in those animals in which the jugular vein catheter became clogged. These animals were injected via the tail vein.

TABLE 4

| Parameter | Vehicle | Allopregnanolone | GNX | GNX |
|---|---|---|---|---|
| Dosage (mg/kg) | 0 | 15 mg/ kg | 12mg/ kg | 15 mg/ kg |
| Vehicle | 30% Captisol/ water | 30% Captisol/ water | 30% Captisol/ water | 30% Captisol/ water |
| Drug (mg/ mL) | 0 | 2.5 | 2.5 | 2.5 |
| Dose volume (mL/ kg) | 6 | 6 | 4.8 | 6 |
| Time of administrations after seizure onset (minutes) | 15’ or 60’ | 15’ or 60’ | 15’ or 60’ | 15’ or 60’ |

Seizure Induction, Treatment, and Recording Protocol

The animals were evaluated in 3 consecutive daily sessions. The experimental schedule is shown in TABLE 5. The time of SE onset was determined by observation and recorded, and the animal dosed with the vehicle, allopregnanolone, or ganaxolone either 15 or 60 min post SE-Onset. Rats were observed for behavior changes at approximately 2, 15, 30, 60, 120 and 240 minutes after pilocarpine administration. Each animal was recorded once and euthanized 5 h after treatment.

TABLE 5

| Day | Time | Activity |
|---|---|---|
| 0 | -18 to -20 hrs | Rats are weighed, dosed with LiCl (127 mg/kg, 5 ml/kg) |
| 1 | -3 hrs | Lights ON |
| 1 | -2.5 hrs | Start recording |
| 1 | -0.5 hrs | Administer Scopolamine MeBr (1mg/kg @ 1ml/kg, intraperitoneal injection (IP) |
| 1 | 0 hrs | Administer pilocarpine (50 mg/kg @ 5mL/kg, IP administration) |
| 1 | 40 m - 1 hr | Status epilepticus onset occurs, time recorded |

(continued)

| TABLE 5 | | |
|---|---|---|
| Day | Time | Activity |
| 1 | Treatment | Vehicle, Allopregnanolone or Ganaxolone administered 15 or 60 minutes after status epilepticus onset |
| 1 | End | End recording 5 hours after treatment begins, animals euthanized, terminal blood samples obtained. |

EEG Analysis Pre, During, and Post Seizure

EEG power ($mV^2/Hz$) was analyzed by Fourier analysis (Fast Fourier Transform, FFT) in 1 Hz frequency bins from 1 to 96 Hz using the ICELUS software. (Although the lowest frequency bin is indicated at "0-5 Hz", technically the lowest frequency recorded was 0.3 Hz). Frequency ranges as follows: Delta, 0-5 Hz (0.3 - 4.99 Hz), Theta 5-10 Hz, Beta 10 - 30 Hz, Gamma-30-50 Hz, Gamma-2 50-70 Hz, Gamma-3 70-96 Hz. EEG power analysis consisted of determining the average power over successive 5 minute time periods. FFT amplitudes were log transformed to minimize biasing results by large amplitude low frequency EEG activity. The baseline EEG period from the start of recording to scopolamine administration was used to normalize EEG power across animals, since all the animals should be in a similar activity state during this period.

To normalize the baseline, the log-FFT values for the entire baseline period and across the entire frequency range (0 - 96 Hz) were summed to obtain a single normalization constant $K_{norm}$:

$$K_{norm} = \sum_{f=0}^{96} \square \sum_{t=-120}^{-10} \log(FFT);$$

f= frequency (Hz); t= time (min)

$K_{norm}$ was subtracted from all FFT power values (at each frequency and all time points) for subsequent analysis. This procedure has the effect of making the average of the total baseline EEG power for each animal equal to zero, after which the baseline frequency curves for all animals should closely overlap.

EEG power data was initially averaged over the following activity time periods: Baseline, Scopolamine, Pilocarpine, SE (the time between SE onset and treatment), and Post treatment intervals: 0-15, 15-30, 30-60 min, 1-2 h, 2-3 h and 3-5 h.

Results

TABLE 6 summarizes the treatment effects for allopregnanolone or ganaxolone administered 15 minutes post seizure onset and TABLE 7 summarizes the treatment effects for allopregnanolone or ganaxolone administered 60 minutes post seizure onset.

Regardless of frequency range, the vehicle groups showed little reduction in EEG power from the time of dosing to approximately 2 h post dosing, then EEG power declined towards baseline from 2 - 5 h post dosing. This decline was more pronounced at higher frequency ranges.

Allopregnanolone at 15 mg/kg, 15 minutes post-seizure onset, produced a strong reduction in SE up to 1 h post dosing that gradually abated. At 2 h post dosing, SE in this group was nearly back to the vehicle level. After a 60' dosing delay, there was a strong reduction in SE in the first 15' after dosing in the 0-5 and 5-10 Hz frequency ranges. In this case, SE returned back to the vehicle by 2 h post dosing and remained there. At 5-10 Hz the reduction in SE lasted less than 1 h, and at higher frequencies only about 15'. Statistically, allopregnanolone only reduced SE for the first 15' when dosed at 60' post SE-onset, whereas when dosed at 60', the reduction was seen for 2-3 h below 30 Hz and for up to 1 h above 30 Hz.

Ganaxolone at 12 and 15 mg/kg produced qualitatively similar results, with both dose levels producing a very strong reduction in SE amplitude overall.

When dosed 15 minutes after SE-onset, GNX reduced EEG power across all frequency ranges to levels at or below baseline for at up to 5 h. From 5-70 Hz, EEG power was reduced to near the baseline level and remained there for 5 h post dosing. At 0-5 Hz, EEG power was reduced below the baseline level for approximately 90 min, and at 70-96 Hz, EEG power remained below baseline for the entire 5 h. However, between 2 to 5 h, EEG power above 30 Hz was not different from the vehicle group, indicating it was also not significantly different from the baseline EEG power level. Note also that at the higher frequency ranges, EEG power in the vehicle groups returned towards baseline after 2 h, so

that differences between the GNX and vehicle groups were not significant.

When dosed at 60' post SE-onset, EEG power was also strongly reduced, but not below the baseline level for frequencies up to 70 Hz. From 70-96 Hz, EEG power appeared to drop below baseline from 1 to 4 h post dosing. The principal difference between effects of GNX dosed at 60' versus 15' post onset were (1) at higher frequencies (> 30 Hz), there were no significant reductions in the first 15' post dosing, and (2) the activity the drug appeared to be stronger especially at higher frequencies at the 2-3 h time point. Again, the EEG power levels were maintained at baseline for up to 5 h post dosing, however, the EEG levels of the vehicle group also returned to baseline for the frequency ranges above 30 Hz so the drug showed no effect. In contrast, at 0-5 Hz, EEG power remained high in the vehicle group, and GNX showed a significant reduction in the 12 mg/kg group.

| TABLE 6 - Treatment Effects, Rats Treated 15 minutes post SE onset | | | | | | | |
|---|---|---|---|---|---|---|---|
| | | Significance, Time post drug administration | | | | | |
| Comparison | Frequency | 0-15 min. | 15-30 | 30-60 | 1-2 hrs | 2-3 hrs | 3-5 hrs |
| Vehicle vs. Allo.(15 mg/ml), both administered 15' post SE onset | 0-5 Hz | **** | **** | **** | *** | * | ns |
| | 5-10 Hz | **** | **** | **** | *** | ns | ns |
| | 10-30 Hz | **** | **** | *** | ** | ns | ns |
| | 30-50 Hz | **** | **** | *** | ns | ns | ns |
| | 50-70 Hz | *** | *** | ** | ns | ns | ns |
| | 70-96 Hz | *** | *** | ** | ns | ns | ns |
| Vehicle vs GNX (12 mg/ml) both administered 15 minutes post SE onset | 0-5 Hz | **** | **** | **** | **** | *** | ** |
| | 5-10 Hz | **** | **** | *** | ** | * | ns |
| | 10-30 Hz | *** | **** | ** | * | ns | ns |
| | 30-50 Hz | *** | *** | ** | * | ns | ns |
| | 50-70 Hz | ** | ** | * | ** | ns | ns |
| | 70-96 Hz | ** | ** | * | ** | ns | ns |
| Vehicle vs GNX (15 mg/ml) both administered 15 minutes post SE onset | 0-5 Hz | **** | **** | **** | **** | **** | *** |
| | 5-10 Hz | **** | **** | **** | **** | *** | ns |
| | 10-30 Hz | **** | **** | **** | ** | * | ns |
| | 30-50 Hz | *** | *** | *** | ** | ns | ns |
| | 50-70 Hz | ** | ** | ** | ** | ns | ns |
| | 70-96 Hz | ** | *** | ** | ** | ns | ns |

| TABLE 7 - Treatment Effects, Rats Treated 60 minutes post SE onset | | | | | | | |
|---|---|---|---|---|---|---|---|
| | | Significance, Time post drug administration | | | | | |
| Comparison | Frequency | 0-15 min. | 15-30 | 30-60 | 1-2 hrs | 2-3 hrs | 3-5 hrs |
| Vehicle vs. Allo.(15 mg/ml), both administered 15' post SE onset | 0-5 Hz | ** | ns | Ns | ns | ns | ns |
| | 5-10 Hz | ** | ns | Ns | ns | ns | ns |
| | 10-30 Hz | ns | ns | Ns | ns | ns | ns |
| | 30-50 Hz | ns | ns | Ns | ns | ns | ns |
| | 50-70 Hz | ns | ns | Ns | ns | ns | ns |
| | 70-96 Hz | ns | ns | Ns | ns | ns | ns |

(continued)

| TABLE 7 - Treatment Effects, Rats Treated 60 minutes post SE onset | | | | | | | | |
|---|---|---|---|---|---|---|---|---|
| | | Significance, Time post drug administration | | | | | | |
| Comparison | Frequency | 0-15 min. | 15-30 | 30-60 | 1-2 hrs | 2-3 hrs | 3-5 hrs |
| Vehicle vs GNX (12 mg/ml) both administered 15 minutes post SE onset | 0-5 Hz | *** | **** | **** | **** | *** | ** |
| | 5-10 Hz | **** | **** | **** | *** | ** | ns |
| | 10-30 Hz | * | ** | ** | ** | * | ns |
| | 30-50 Hz | ns | ns | * | ** | * | ns |
| | 50-70 Hz | ns | * | ** | ** | * | ns |
| | 70-96 Hz | ns | ** | *** | *** | ** | ns |
| Vehicle vs GNX (15 mg/ml) both administered 15 minutes post SE onset | 0-5 Hz | *** | *** | ** | ** | ** | ns |
| | 5-10 Hz | ** | **** | **** | ** | ** | ns |
| | 10-30 Hz | ns | ns | * | ** | * | ns |
| | 30-50 Hz | ns | ns | * | * | ns | ns |
| | 50-70 Hz | ns | ns | * | ** | ns | ns |
| | 70-96 Hz | ns | * | ** | ** | * | ns |
| * P < 0.05; ** P < 0.01; *** P < 0.001; **** P < 0.0001, ns = not significant CLAIMS | | | | | | | |

Further embodiments of the present invention are described below:

1. An aqueous injectable ganaxolone formulation comprising

a) ganaxolone and sulfobutyl ether-β-cyclodextrin in an inclusion complex; and
b) water.

2. The formulation of embodiment 1, wherein the complex comprising ganaxolone and sulfobutyl ether-β-cyclodextrin comprises a 1:1 ganaxolone: sulfobutyl ether-β-cyclodextrin complex.
3. The formulation of embodiment 1 or 2, wherein the ganaxolone concentration is about 0.1 mg/mL to about 15 mg/mL.
4. The formulation of any one of embodiments 1-3, additionally comprising a 0.5 % to 1.5% concentration of sodium chloride in the formulation.
5. The formulation of any one of embodiments 1-4, wherein the w/w ratio of sulfobutyl ether-β-cyclodextrin to ganaxolone is about 52:1 or greater.
6. The formulation of any one of embodiments 1-4, wherein the w/w ratio of sulfobutyl ether-β-cyclodextrin to ganaxolone is within the range from about 52:1 to about 80:1.
7. The formulation of embodiment 6, wherein the w/w ratio of sulfobutyl ether-β-cyclodextrin to ganaxolone is about 55:1.
8. The formulation of any one of embodiments 1-7, comprising 5 mg/ mL to 800 mg/ml sulfobutyl ether-β-cyclodextrin.
9. The formulation of any one of embodiment 1-8, comprising a surfactant.
10. The formulation of embodiment 9, wherein the surfactant is a sorbitan ester, a polyoxyethylene sorbitan fatty acid ester, a poloxamer, a cholesterol salt, or a bile salt.
11. The formulation of embodiment 10, wherein the formulation is about 0.5 to about 15 weight percent surfactant.
12. The formulation of embodiment 11, wherein the surfactant is polysorbate 80.
13. The formulation of any one of embodiments 1-12, having a pH in the range of approximately 2.5-11.0.
14. The formulation of any one of embodiments 1-13, additionally comprising a buffer.
15. The formulation of embodiment 14, having a pH of about 6.8 to about 7.6.
16. The formulation of embodiment 14 or 15, wherein the buffer is a phosphate buffer.
17. The formulation of embodiment 16, wherein the buffer is phosphate buffered saline.
18. The formulation of embodiment 16, wherein the buffer is a combination of a monobasic phosphate buffer and

a dibasic phosphate buffer, wherein the concentration of each phosphate buffer is 2 mM to 50 mM.

19. The formulation of embodiment 1, wherein

the concentration of ganaxolone is 2 mg/ ml to 8 mg/ ml,
the w/w ratio of sulfobutyl ether-β-cyclodextrin to ganaxolone is within the range from about 52:1 to about 90:1;
the formulation contains a buffer and has a pH of 6.7 to 7.3; and
the formulation contains from 0.5 to 15 weight percent surfactant.

20. The formulation of embodiment 1, wherein

the concentration of ganaxolone is 1 mg/ ml to 5 mg/ ml;
the weight percent of sulfobutyl ether-β-cyclodextrin 25% to 35%; and
the formulation contains from 5% to 15% (weight percent) of at least one of the following: a surfactant, ethanol, glycerin or propylene glycol.

21. The formulation of any one of embodiments 1 to 19, additionally comprising a preservative.

22. The formulation of embodiment 20, wherein the preservative is benzyl alcohol, chlorbutanol, 2-ethoxyethanol, parabens (including methyl, ethyl, propyl, butyl, and combinations), benzoic acid, sorbic acid, chlorhexidene, phenol, 3-cresol, thimerosal, or a phenylmercurate salt.

23. The formulation of embodiment 22, wherein the inclusion complex provides at least 2.0 mg/ mL ganaxolone, when the amount of ganaxolone provided by the complex is measured at a sulfobutyl ether-β-cyclodextrin concentration of about 30% w/v in water.

24. A lyophilized ganaxolone formulation comprising ganaxolone and sulfobutyl ether-β-cyclodextrin, wherein the ganaxolone formulation is 1.0% to 1.5% ganaxolone.

25. A lyophilized ganaxolone formulation comprising the ganaxolone formulation of any one of embodiments 1 to 23.

26. The lyophilized ganaxolone formulation of embodiment 25, additionally comprising one or more of a surfactant, a buffer, and a preservative.

27. The lyophilized ganaxolone formulation of any one of embodiments 24 to 26, wherein the lyophilized ganaxolone formulation can be reconstituted in water to provide a clear solution.

28. The lyophilized ganaxolone formulation of embodiment 27, additionally comprising a bulking agent.

29. The lyophilized ganaxolone formulation of embodiment 28, wherein the bulking agent is mannitol, lactose, sucrose, trehalose, sorbitol, glucose, rafinose, glycine, histidine, polyethylene glycol (PEG), or polyvinyl pyrrolidone (PVP).

30. A method of treating a patient having a seizure disorder, stroke, or traumatic brain injury, the method comprising administering a therapeutically effective amount of the ganaxolone formulation of any one of embodiments 1 to 29.

31. The method of embodiment 30, wherein the seizure disorder is status epilepticus, refractory status epilepticus, super refractory status epilepticus, or PCDH19 female pediatric epilepsy.

32. The method of embodiment 30 or 31 wherein the amount of ganaxolone administered is from about 1 mg/kg to about 200 mg/kg.

33. The method of any one of embodiments 30 to 32 wherein the ganaxolone formulation is administered intramuscularly or intravenously.

34. The method of any one of embodiments 30 to 33 comprising administering a single bolus dose of the ganaxolone formulation to the patient.

35. The method of embodiment 34 wherein the bolus dose provides a sufficient amount of ganaxolone to provide a plasma $C_{max}$ of ganaxolone of about 100 ng/ mL to about 1000 ng/ mL in the patient.

36. The method of embodiment 34, wherein the bolus dose provides a sufficient amount of ganaxolone to provide a plasma $C_{max}$ of ganaxolone of about 600 ng/ mL to about 900 ng/ mL in the patient.

37. The method of any one of embodiments 34 to 36, wherein the bolus dose in administered in less than 10 minutes and Cmax occurs within 1 hour of completion of administration.

38. The method of embodiment 34, wherein the single bolus dose comprises from about 1 mg/kg to about 20 mg/kg ganaxolone.

39. The method of any one of embodiments 30 to 33 comprising administering multiple bolus doses of the ganaxolone formulation to the patient.

40. The method of embodiment 39 wherein the multiple bolus doses are given over 1 to 10 days at intervals of 1 to 24 hours.

41. The method of embodiment 39 wherein each bolus dose provides a sufficient amount of ganaxolone to produce a plasma $C_{max}$ of ganaxolone of about 100 ng/ mL to about 1000 ng/ mL in the patient.

42. The method of embodiment 41, wherein the interval between bolus doses is from about 10 to about 24 hours

and once an initial $C_{max}$ is reached the plasma concentration of ganaxolone is not below 100 ng/ mL at any time between bolus doses.

43. The method of embodiment 41, wherein the interval between bolus doses is from about 20 to about 24 hours and once an initial $C_{max}$ is reached the concentration of ganaxolone in the patient's plasma does not fall below 25% of the initial $C_{max}$ at any time between bolus doses.

44. The method of any one of embodiments 39 to 43 wherein each bolus dose comprises about 1 mg/kg to about 20 mg/kg ganaxolone.

45. The method of any one of embodiments 30 to 32 comprising administering an intravenous infusion of the ganaxolone formulation to the patient, with or without an initial bolus dose.

46. The method of embodiment 45 comprising administering the intravenous infusion for 1 to 10 consecutive days at a rate of 1 to 10 mg/kg/hr without an initial bolus dose.

47. The method of embodiment 45 comprising administering an initial bolus dose of from about 1 mg/kg to about 20 mg/kg ganaxolone, followed within 24 hours by administration of an intravenous infusion of the ganaxolone formulation for 1 to 10 consecutive days at a rate of 1 to 10 mg/kg/hr.

48. The method of embodiment 47, wherein the initial bolus dose provides a sufficient amount of ganaxolone to provide an initial plasma $C_{max}$ of ganaxolone of about 100 ng/ mL to about 1000 ng/ mL in the patient and the concentration of ganaxolone in the patient's plasma does not fall below 25% of the initial $C_{max}$ until after the infusion is concluded.

49. The method of embodiment 47, wherein the initial bolus dose provides a sufficient amount of ganaxolone to provide an initial plasma $C_{max}$ of ganaxolone of about 100 ng/ mL to about 1000 ng/ mL in the patient, the patient is then administered an intravenous infusion of the ganaxolone formulation at a constant dose sufficient to provide a concentration of ganaxolone in the patient's plasma of at least 40 % of $C_{max}$, followed by an intravenous infusion of ganaxolone formulation at a gradually reducing dose so that the concentration of ganaxolone in the patient's plasma is less than 20% of $C_{max}$ when the intravenous infusion is concluded.

50. The method any one of embodiments 30 to 49 wherein the ganaxolone formulation is a first active agent and is administered concurrently or sequentially with at least one additional active agent.

51. The method of embodiment 50 wherein the at least one additional active agent is an anticonvulsant or anesthetic/sedative.

52. The method of embodiment 51 wherein the at least one additional active agent is an anticonvulsant chosen from a $GABA_A$ receptor modulator, a sodium channel blocker, a GAT-1 GABA transporter modulator, a GABA transaminase modulator, a voltage-gated calcium channel blocker, and a peroxisome proliferator-activated alpha modulator.

53. The method of embodiment 51 wherein the at least one additional active agent is an anesthetic/sedative chosen from an inhalational anesthetics (including desflurane, enflurane, ethyl chloride, halothane, isoflurane, methoxyflurane, sevoflurane, and trichloroethylene), an intravenous, non-barbiturate anesthetics (including atracurium, cisatracurium, etodimidate, ketamine, propofol, and rocuronium), a barbiturate anesthetic (including amobarbital, methohexital, pentobarbital, phenobarbital, secobarbital, thiamylal, and thiopental), and a benzodiazepine anesthetic (including diazepam, flunitrazepam, lorazepam, and midazolam).

54. The method of embodiment 51, wherein the additional active agent is an anesthetic/ sedative and the patient is given a sufficient dosage of the anesthetic/ sedative to induce coma.

55. The method of embodiment 54, wherein the additional active agent is a barbiturate.

56. The method of embodiment 54, wherein the additional active agent is pentobarbital or thiopental.

57. The method of embodiment 54, wherein the additional active agent is propofol.

58. The method of embodiment 50, wherein a first additional active agent is an anticonvulsant and a second additional active agent is an anesthetic/sedative.

59. The method of embodiment 58, wherein the anticonvulsant is carbamazepine, tiagabine, levetiracetam, lamotrigine, pregabalin, gabapentin, or phenytoin and the anesthetic/sedative is pentobarbital, thiopental, or propofol.

## Claims

**1.** An aqueous injectable ganaxolone formulation comprising

a) ganaxolone and sulfobutyl ether-β-cyclodextrin in an inclusion complex; and
b) water, wherein the w/w ratio of sulfobutyl ether-β-cyclodextrin to ganaxolone is from 52:1 to 80:1.

**2.** The formulation of claim 1, wherein the ganaxolone concentration is from 0.1 mg/ml to 15 mg/ml, preferably from 1 mg/ml to 10 mg/ml.

3. The formulation of any preceding claim, wherein the ganaxolone concentration is 1 mg/ml.

4. The formulation of any preceding claim, wherein the w/w ratio of sulfobutyl ether-β-cyclodextrin to ganaxolone is from 55:1 to 65:1.

5. The formulation of any preceding claim, additionally comprising a tonicity adjusting agent, optionally wherein the tonicity adjusting agent is sodium chloride.

6. The formulation of any preceding claim, additionally comprising a buffer.

7. The formulation of claim 6, wherein the buffer is citrate.

8. The formulation of claim 6, wherein the buffer is histidine.

9. The formulation of claim 6, wherein the buffer is a phosphate, optionally wherein the buffer is phosphate buffered saline.

10. The formulation of claim 6, wherein the buffer is a combination of a monobasic phosphate buffer and a dibasic phosphate buffer, wherein the concentration of each phosphate buffer is from 2 mM to 50 mM, preferably from 5 mM to about 20 mM.

11. The formulation of any one of claims 6-10, having a pH of 6.0 to 8.0.

12. An aqueous injectable ganaxolone formulation comprising

a) ganaxolone and sulfobutyl ether-β-cyclodextrin in an inclusion complex; and
b) water, wherein the w/w ratio of sulfobutyl ether-β-cyclodextrin to ganaxolone is from 52:1 to 80:1, and wherein the concentration of ganaxolone is from 1 mg/ml to 10 mg/ml; and
c) a buffer; and having a pH of 6.0 to 8.0.

13. An aqueous injectable ganaxolone formulation of any preceding claim for use in a method of treating a patient having a seizure disorder, stroke, or traumatic brain injury.

14. The aqueous injectable ganaxolone formulation for use of claim 13, wherein the seizure disorder is status epilepticus.

FIGURE 1.

Ganaxolone concentration in solution vs. CAPTISOL concentration added

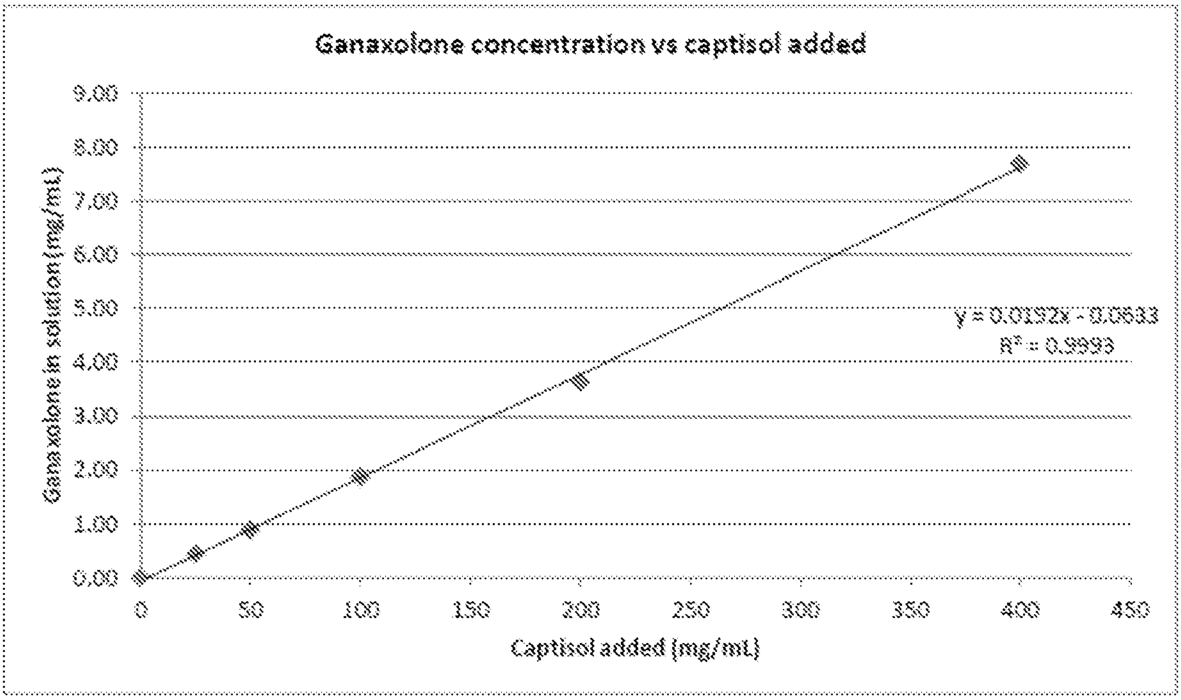

FIGURE 2.

Plasma Exposure of Ganaxolone in Rats Infused with Ganaxolone-CAPTISOL Solution

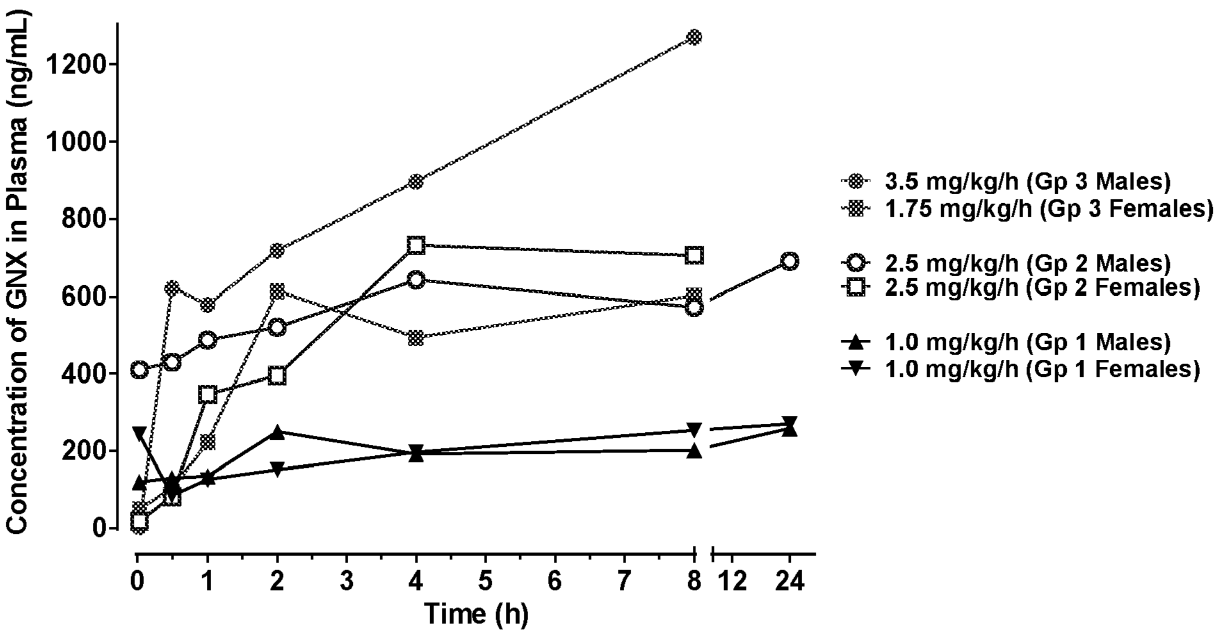

FIGURE 3.

EEG Power vs. Time for Drug (Vehicle, Ganaxolone or Allopregnanolone) Administered 15 Minutes After SE Onset

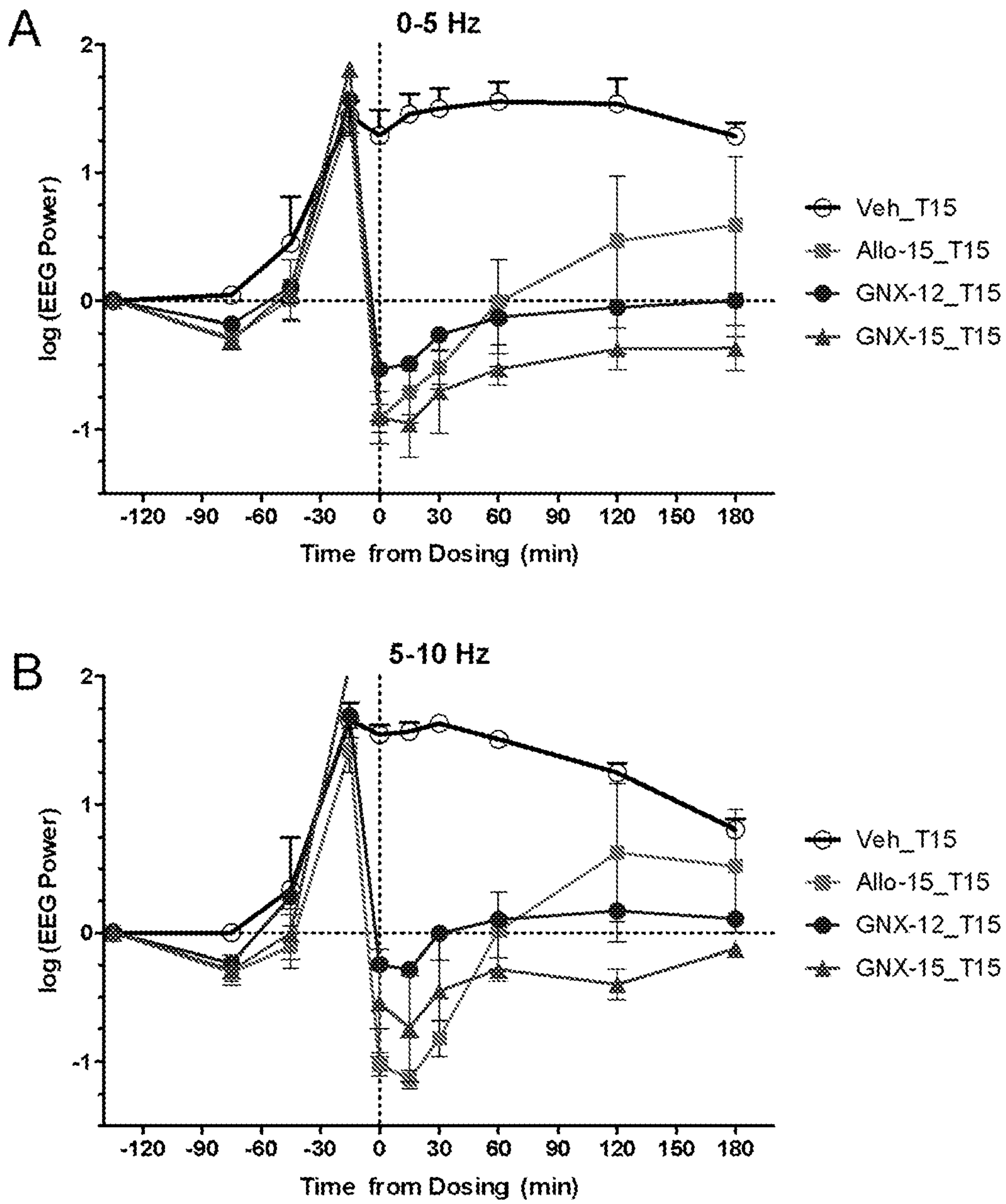

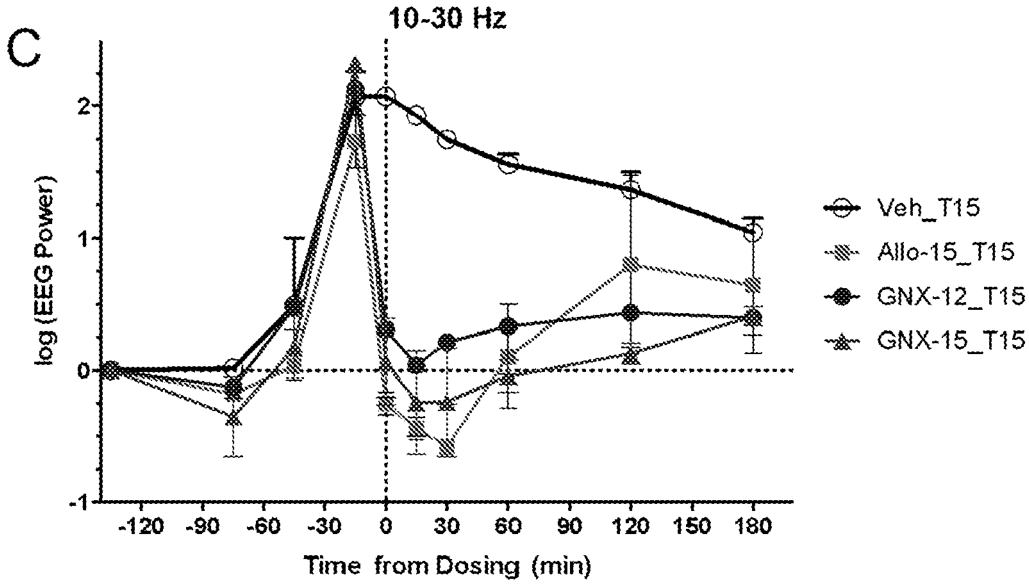
C
10-30 Hz
log (EEG Power)
Time from Dosing (min)
Veh_T15
Allo-15_T15
GNX-12_T15
GNX-15_T15

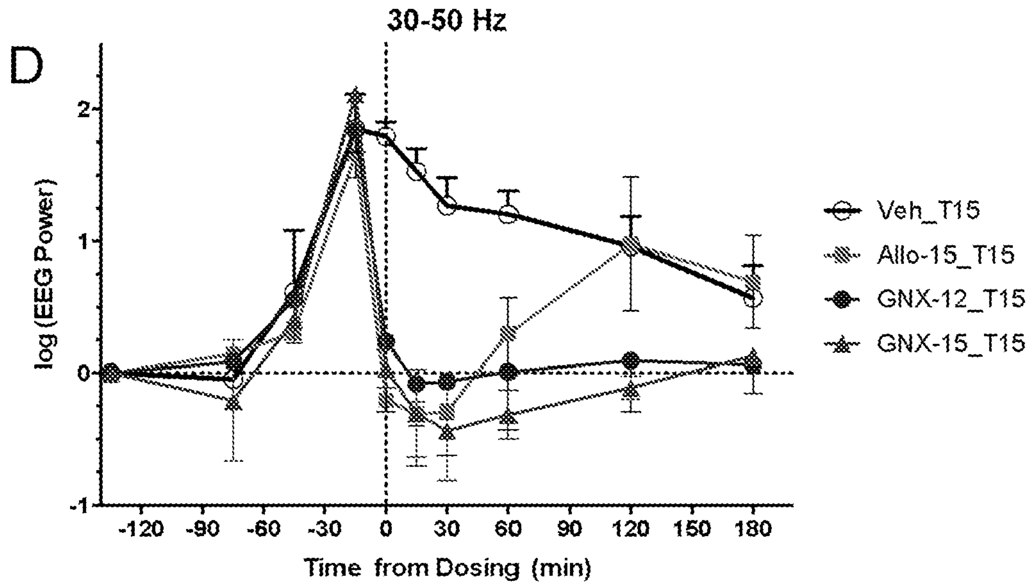
D
30-50 Hz
log (EEG Power)
Time from Dosing (min)
Veh_T15
Allo-15_T15
GNX-12_T15
GNX-15_T15

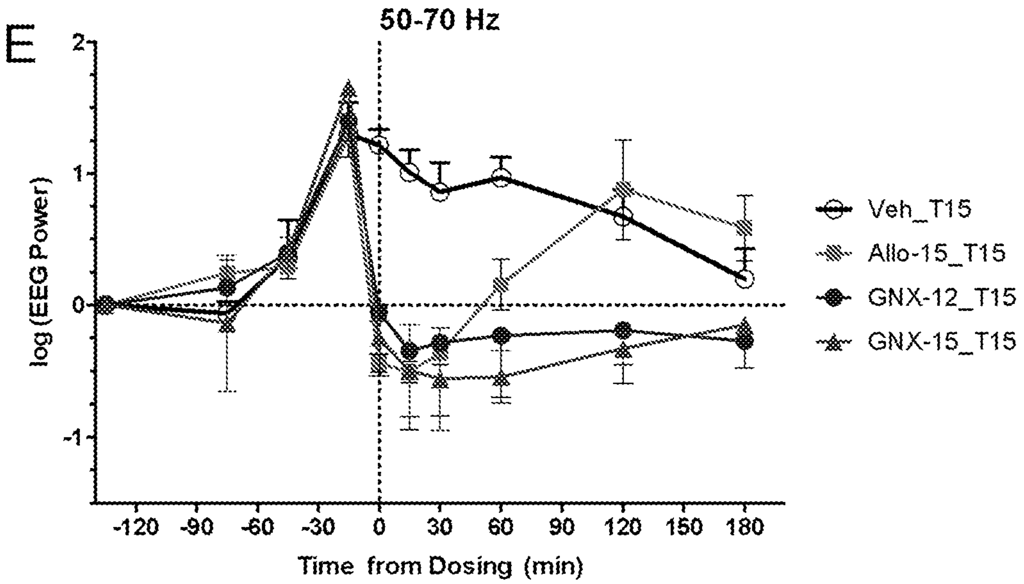
E
50-70 Hz
log (EEG Power)
2
1
0
-1
-120 -90 -60 -30 0 30 60 90 120 150 180
Time from Dosing (min)
Veh_T15
Allo-15_T15
GNX-12_T15
GNX-15_T15

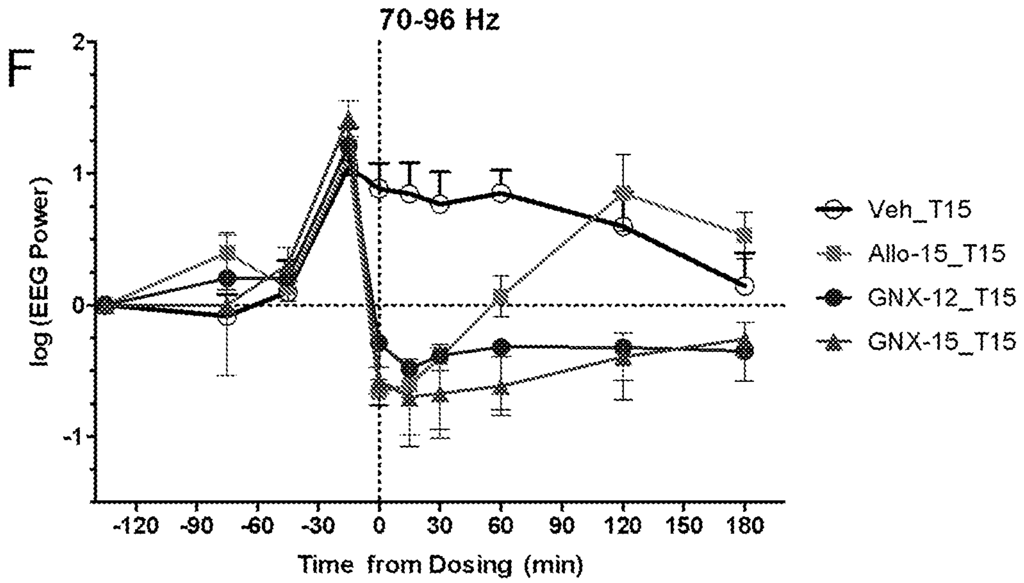
F
70-96 Hz
log (EEG Power)
2
1
0
-1
-120 -90 -60 -30 0 30 60 90 120 150 180
Time from Dosing (min)
Veh_T15
Allo-15_T15
GNX-12_T15
GNX-15_T15

FIGURE 4.

EEG Power vs. Time for Drug (Vehicle, Ganaxolone or Allopregnanolone) Administered 60 Minutes After SE Onset

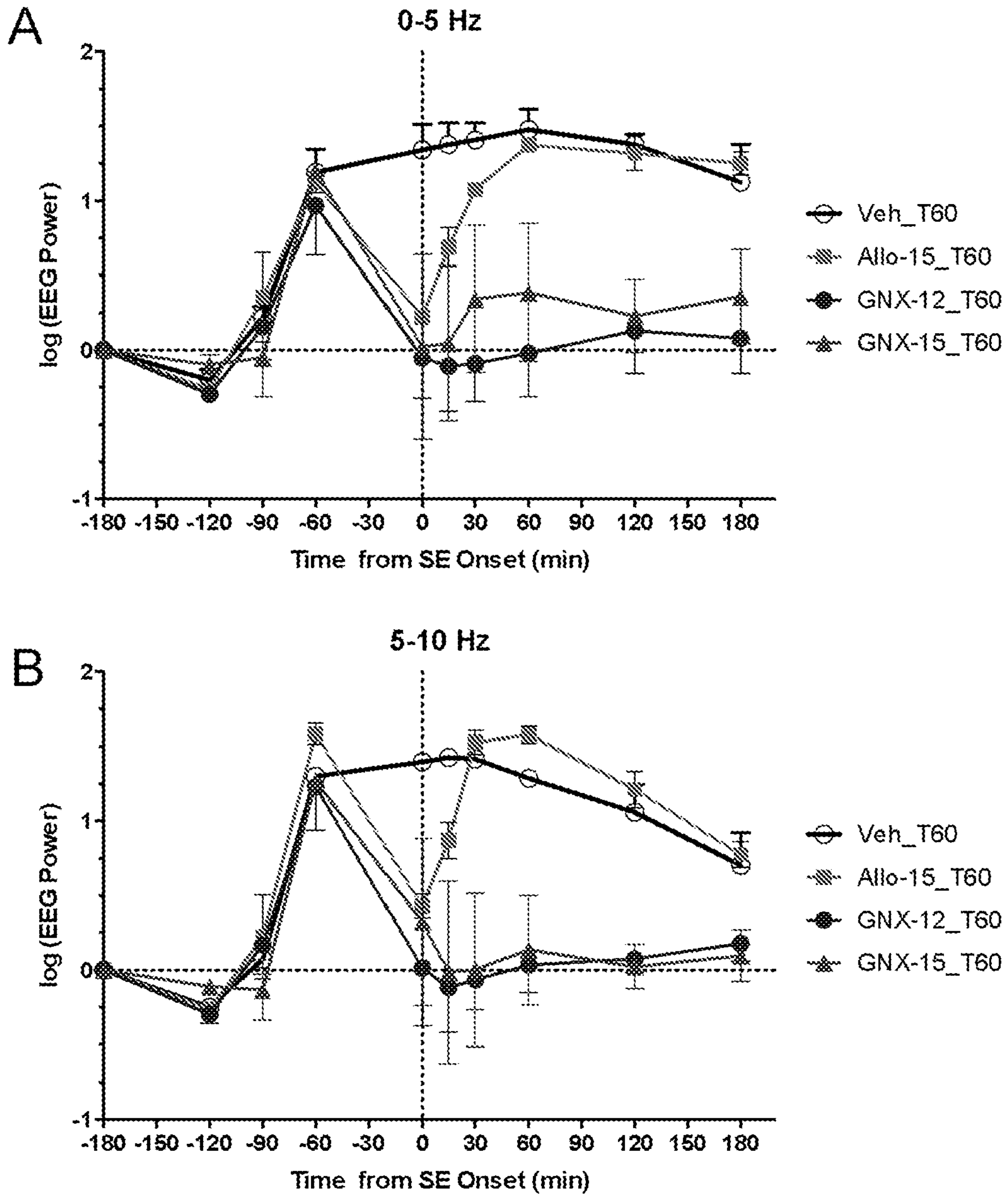

C

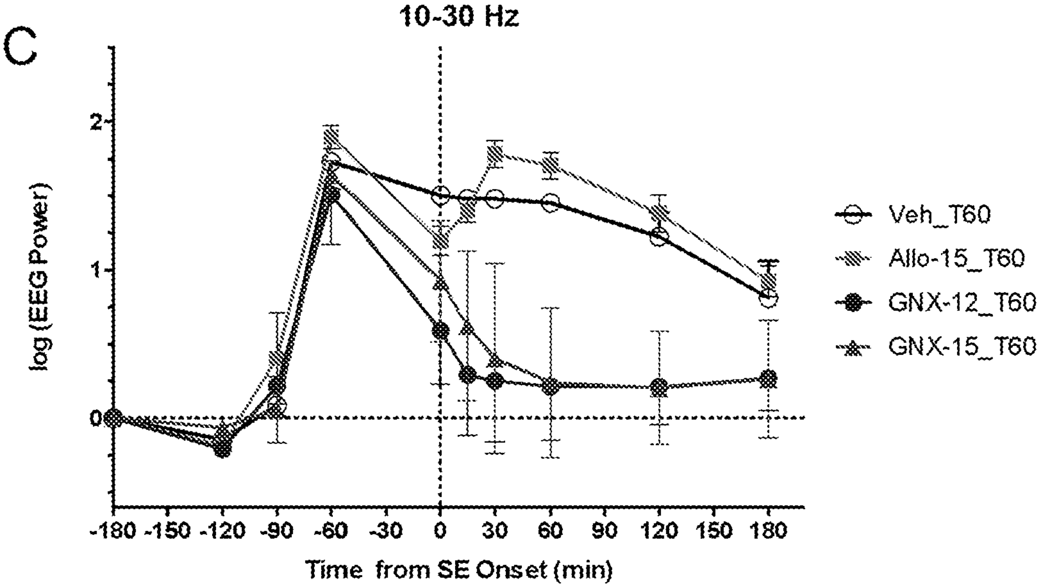
10-30 Hz
log (EEG Power)
2
1
0
-180 -150 -120 -90 -60 -30 0 30 60 90 120 150 180
Time from SE Onset (min)
Veh_T60
Allo-15_T60
GNX-12_T60
GNX-15_T60

D

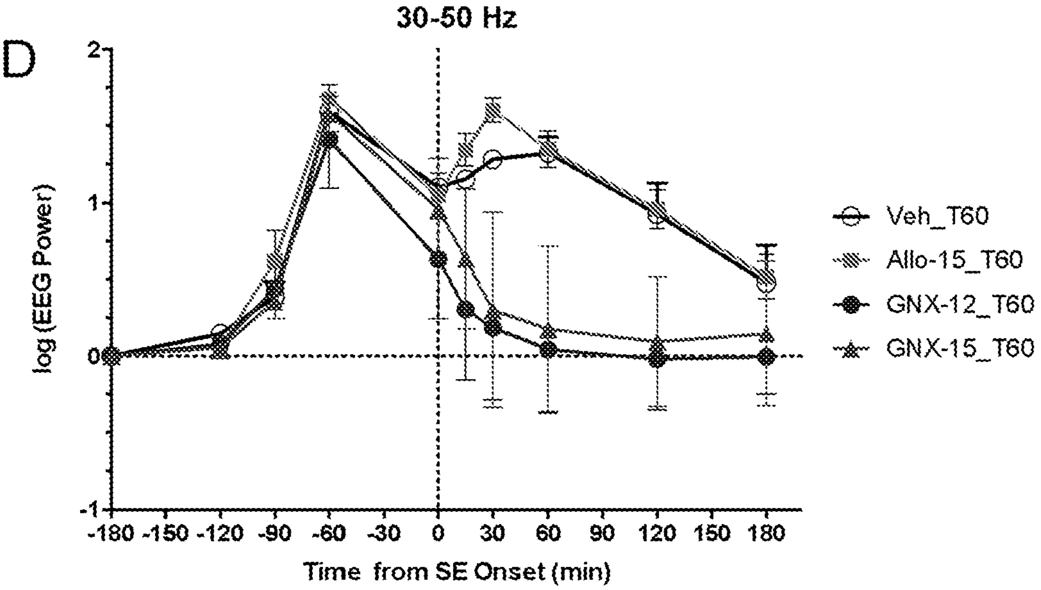
30-50 Hz
log (EEG Power)
2
1
0
-1
-180 -150 -120 -90 -60 -30 0 30 60 90 120 150 180
Time from SE Onset (min)
Veh_T60
Allo-15_T60
GNX-12_T60
GNX-15_T60

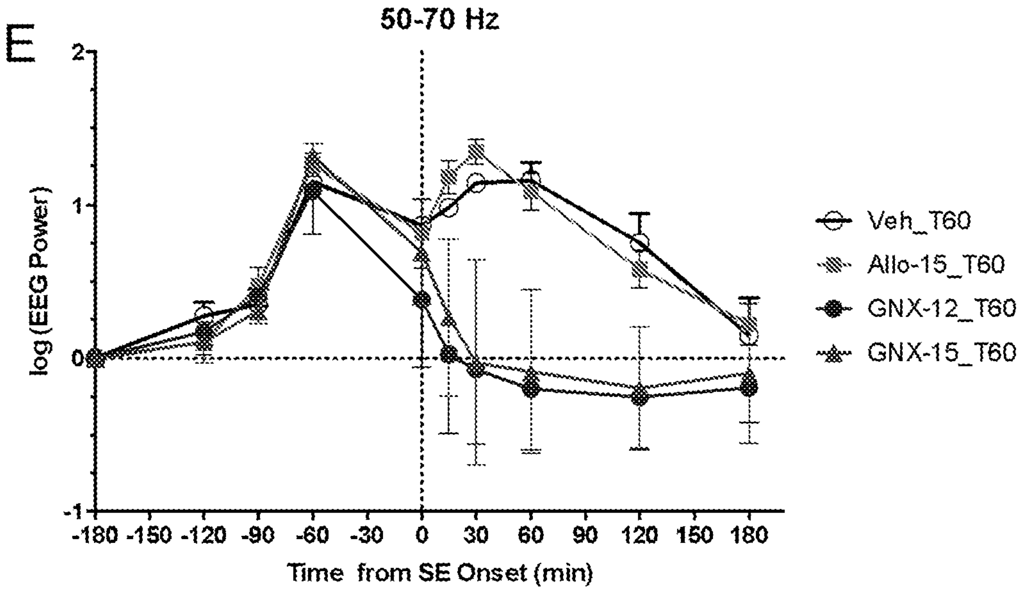
50-70 Hz
E
log (EEG Power)
2
1
0
-1
-180 -150 -120 -90 -60 -30 0 30 60 90 120 150 180
Time from SE Onset (min)
Veh_T60
Allo-15_T60
GNX-12_T60
GNX-15_T60

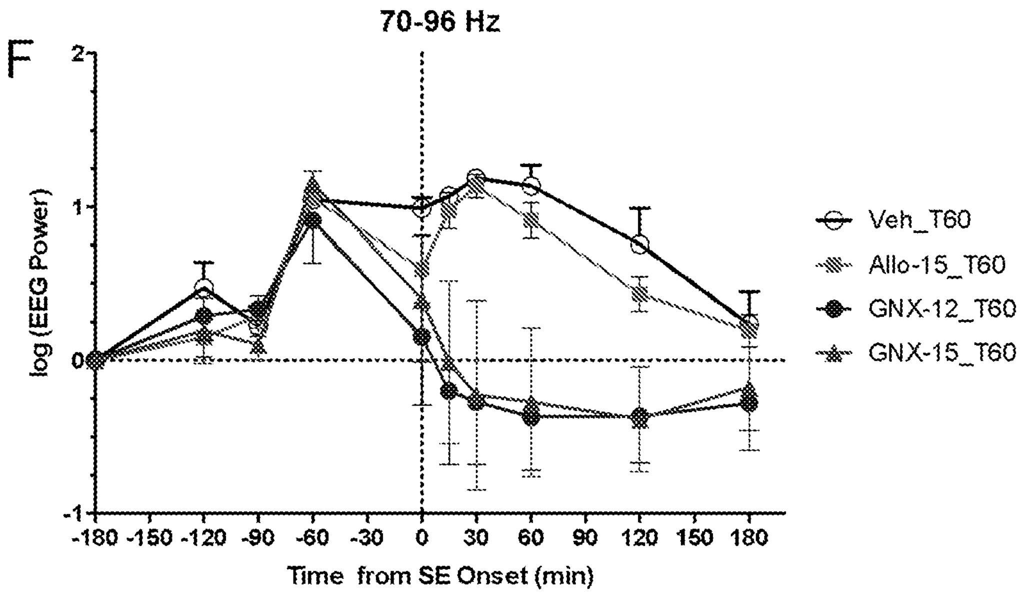
70-96 Hz
F
log (EEG Power)
2
1
0
-1
-180 -150 -120 -90 -60 -30 0 30 60 90 120 150 180
Time from SE Onset (min)
Veh_T60
Allo-15_T60
GNX-12_T60
GNX-15_T60

Europäisches Patentamt
European Patent Office
Office européen des brevets

# EUROPEAN SEARCH REPORT

Application Number
EP 22 15 2108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2011/088503 A1 (GOODCHILD INVEST PTY LTD [AU]; UNIV MONASH [AU]; GOODCHILD JULIET MARG) 28 July 2011 (2011-07-28)<br>* paragraphs [0055], [0067], [0072], [0073], [0122], [0127] *<br>* examples 1,8,9 *<br>----- | 1-12<br><br><br>1-14 | INV.<br>A61K47/69<br>A61K31/57<br>A61K9/00<br>A61P25/08 |
| X<br><br><br>Y | WO 2014/085668 A1 (UNIV CALIFORNIA [US]; ROGAWSKI MICHAEL A [US]; ZOLKOWSKA DOROTA [US]) 5 June 2014 (2014-06-05)<br>* paragraphs [0007], [0008], [0020], [0075], [0080] *<br>* figure 1 *<br>----- | 13,14<br><br><br>1-14 | |
| A | LIPTAKOVA S ET AL: "EFFECT OF GANAXOLONE ON FLUROTHYL SEIZURES IN DEVELOPING RATS", EPILEPSIA, RAVEN PRESS LTD, NEW YORK, US, vol. 41, no. 7, 1 January 2000 (2000-01-01), pages 788-793, XP009054309, ISSN: 0013-9580, DOI: 10.1111/J.1528-1157.2000.TB00244.X<br>* abstract *<br>----- | 1-14 | TECHNICAL FIELDS SEARCHED (IPC)<br>A61K<br>A61P |
| A | Marinus Pharmaceuticals: "Marinus Pharmaceuticals, Inc. Enters Into Use Agreement With CyDex Pharmaceuticals, Inc. for Use of Captisol(R) for Ganaxolone IV", ,<br>12 August 2014 (2014-08-12), XP002756531, Retrieved from the Internet: URL:http://ir.marinuspharma.com/releasedetail.cfm?releaseid=865715 [retrieved on 2016-04-14]<br>* the whole document *<br>-----<br>-/-- | 1-14 | |

The present search report has been drawn up for all claims

1

| Place of search | Date of completion of the search | Examiner |
|---|---|---|
| The Hague | 9 August 2022 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 2108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | WO 2016/040322 A1 (SAGE THERAPEUTICS INC [US]; KANES STEPHEN JAY [US]; COLQUHOUN HELEN [U) 17 March 2016 (2016-03-17)<br>* page 40, last paragraph *<br>* page 64, last paragraph *<br>* page 73, last paragraph *<br>----- | 1-14 | |
| T | LOFTSSON THORSTEINN ET AL: "Summary", JOURNAL OF PHARMACY AND PHARMACOLOGY : JPP,<br>vol. 68, no. 5, 9 June 2015 (2015-06-09), pages 544-555, XP055796310,<br>GB<br>ISSN: 0022-3573, DOI: 10.1111/jphp.12427<br>Retrieved from the Internet:<br>URL:https://api.wiley.com/onlinelibrary/tdm/v1/articles/10.1111%2Fjphp.12427><br>* page 544, right-hand column *<br>----- | 1-14 | |
| T | ARORA ANUREET ET AL: "Inclusion complexes of atorvastatin calcium-sulfobutyl ether [beta] cyclodextrin with enhanced hypolipidemic activity",<br>JOURNAL OF APPLIED PHARMACEUTICAL SCIENCE, vol. 9, no. 11,<br>1 November 2019 (2019-11-01), pages 60-68, XP055871398,<br>DOI: 10.7324/JAPS.2019.91108<br>Retrieved from the Internet:<br>URL:https://pdfs.semanticscholar.org/3d76/f8ba5123d8fcf1836c2f88a6b509fcd57e75.pdf><br>* page 61, left-hand column, penultimate paragraph *<br>-----<br>-/-- | 1-14 | TECHNICAL FIELDS SEARCHED (IPC) |

The present search report has been drawn up for all claims

| Place of search | Date of completion of the search | Examiner |
|---|---|---|
| The Hague | 9 August 2022 | Villard, Anne-Laure |

1

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 2 of 3

Europäisches Patentamt
European Patent Office
Office européen des brevets

# EUROPEAN SEARCH REPORT

Application Number
EP 22 15 2108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MULA MARCO: "Emerging drugs for focal epilepsy.", EXPERT OPINION ON EMERGING DRUGS MAR 2013, vol. 18, no. 1, March 2013 (2013-03), pages 87-95, XP002756532, ISSN: 1744-7623<br>* page 90, section 6.1 *<br>----- | 1-14 | |
| | | | TECHNICAL FIELDS SEARCHED (IPC) |

1

The present search report has been drawn up for all claims

| Place of search | Date of completion of the search | Examiner |
|---|---|---|
| The Hague | 9 August 2022 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 22 15 2108

This annex lists the patent family members relating to the patent documents cited in the above-mentioned European search report. The members are as contained in the European Patent Office EDP file on
The European Patent Office is in no way liable for these particulars which are merely given for the purpose of information.

09-08-2022

| Patent document cited in search report | Publication date | Patent family member(s) | Publication date |
|---|---|---|---|
| WO 2011088503 A1 | 28-07-2011 | AU 2011207103 A1 | 26-07-2012 |
| | | BR 112012017800 A2 | 10-07-2018 |
| | | CA 2786762 A1 | 28-07-2011 |
| | | CL 2012002032 A1 | 21-12-2012 |
| | | CN 102802635 A | 28-11-2012 |
| | | CY 1119947 T1 | 12-12-2018 |
| | | DK 2525798 T3 | 20-11-2017 |
| | | EP 2525798 A1 | 28-11-2012 |
| | | ES 2646829 T3 | 18-12-2017 |
| | | GB 2484244 A | 04-04-2012 |
| | | GB 2491491 A | 05-12-2012 |
| | | HK 1169031 A1 | 18-01-2013 |
| | | HR P20171699 T1 | 26-01-2018 |
| | | HU E035441 T2 | 02-05-2018 |
| | | JP 5930311 B2 | 08-06-2016 |
| | | JP 2013517299 A | 16-05-2013 |
| | | KR 20120136347 A | 18-12-2012 |
| | | LT 2525798 T | 10-01-2018 |
| | | NO 2525798 T3 | 06-01-2018 |
| | | NZ 601255 A | 27-09-2013 |
| | | PL 2525798 T3 | 30-05-2018 |
| | | PT 2525798 T | 15-11-2017 |
| | | RU 2012134321 A | 27-02-2014 |
| | | SG 181997 A1 | 30-08-2012 |
| | | SI 2525798 T1 | 31-01-2018 |
| | | US 2012316146 A1 | 13-12-2012 |
| | | US 2014066417 A1 | 06-03-2014 |
| | | WO 2011088503 A1 | 28-07-2011 |
| | | ZA 201205370 B | 25-09-2013 |
| WO 2014085668 A1 | 05-06-2014 | AU 2013352141 A1 | 18-06-2015 |
| | | AU 2018204865 A1 | 19-07-2018 |
| | | AU 2020201919 A1 | 02-04-2020 |
| | | AU 2022202943 A1 | 26-05-2022 |
| | | CA 2892811 A1 | 05-06-2014 |
| | | EP 2925327 A1 | 07-10-2015 |
| | | JP 6966981 B2 | 17-11-2021 |
| | | JP 2016501876 A | 21-01-2016 |
| | | JP 2018193377 A | 06-12-2018 |
| | | JP 2021152074 A | 30-09-2021 |
| | | US 2015313915 A1 | 05-11-2015 |
| | | US 2018133229 A1 | 17-05-2018 |
| | | US 2018193357 A1 | 12-07-2018 |
| | | US 2019142845 A1 | 16-05-2019 |
| | | US 2021100817 A1 | 08-04-2021 |
| | | WO 2014085668 A1 | 05-06-2014 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO.

EP 22 15 2108

This annex lists the patent family members relating to the patent documents cited in the above-mentioned European search report. The members are as contained in the European Patent Office EDP file on
The European Patent Office is in no way liable for these particulars which are merely given for the purpose of information.

09-08-2022

| Patent document cited in search report | Publication date | Patent family member(s) | Publication date |
|---|---|---|---|
| WO 2016040322 A1 | 17-03-2016 | AU 2015315333 A1 | 23-03-2017 |
| | | AU 2021202433 A1 | 13-05-2021 |
| | | BR 112017004535 A2 | 03-07-2018 |
| | | CA 2960611 A1 | 17-03-2016 |
| | | CN 107106574 A | 29-08-2017 |
| | | CO 2017003396 A2 | 31-08-2017 |
| | | EP 3191101 A1 | 19-07-2017 |
| | | IL 285703 A | 30-09-2021 |
| | | JO 3667 B1 | 27-08-2020 |
| | | JP 6837430 B2 | 03-03-2021 |
| | | JP 2017526703 A | 14-09-2017 |
| | | JP 2020059760 A | 16-04-2020 |
| | | JP 2022113879 A | 04-08-2022 |
| | | KR 20170048567 A | 08-05-2017 |
| | | PE 20170904 A1 | 12-07-2017 |
| | | PE 20221050 A1 | 30-06-2022 |
| | | PH 12017500427 A1 | 31-07-2017 |
| | | RU 2017111816 A | 10-10-2018 |
| | | SG 10202107866Y A | 30-08-2021 |
| | | SG 11201701815S A | 27-04-2017 |
| | | TW 201625266 A | 16-07-2016 |
| | | TW 202106306 A | 16-02-2021 |
| | | US 2017348327 A1 | 07-12-2017 |
| | | US 2020253985 A1 | 13-08-2020 |
| | | WO 2016040322 A1 | 17-03-2016 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5134127 A
- US 5376645 A, Stella


### Non-patent literature cited in the description


- **ROSSETTI, A.O. ; LOWENSTEIN, D.H.** *Lancet Neurol.,* 2011, vol. 10 (10), 922-930
- **SHORVON, S. ; FERLISI, M.** *Brain,* 2011, vol. 134 (10), 2802-2818
- **ROGAWSKI, M.A. et al.** *Epilepsia,* 2013, vol. 54, 93-98
- *CHEMICAL ABSTRACTS,* 38398-32-2
- **NOHRIA, V. ; GILLER, E.** *Neurotherapeutics,* 2007, vol. 4 (1), 102-105
- *CHEMICAL ABSTRACTS,* 9005-64-5
- *CHEMICAL ABSTRACTS,* 9005-65-6